(19) Europäisches Patentamt
European Patent Office
Office européen des brevets

(11) Numéro de publication: **0 226 508**
**A1**

(12) # DEMANDE DE BREVET EUROPEEN

(21) Numéro de dépôt: **86402676.0**

(22) Date de dépôt: **02.12.86**

(51) Int. Cl.[4]: **C 07 D 471/04**
A 61 K 31/435
//(C07D471/04,221:00,209:00)

(30) Priorité: **02.12.85 FR 8518209**
**02.12.85 FR 8518210**

(43) Date de publication de la demande:
**24.06.87 Bulletin 87/26**

(84) Etats contractants désignés:
**AT BE CH DE ES FR GB GR IT LI LU NL SE**

(71) Demandeur: **SANOFI**
**40, Avenue George V**
**F-75008 Paris (FR)**

(72) Inventeur: **Bras, Jean-Pierre**
**34, rue de Rémusat**
**31000 Toulouse (FR)**

**De Cointet, Paul**
**224, Avenue Jean Rieux**
**31500 Toulouse (FR)**

**Pepin, Odile**
**23, rue Saint Eloi**
**31400 Toulouse (FR)**

**Pierre, Alain**
**13 Jardins Occitans**
**F-31520 Ramonville St. Agne (FR)**

(74) Mandataire: **Bressand, Georges et al**
**c/o CABINET LAVOIX 2 Place d'Estienne d'Orves**
**F-75441 Paris Cedex 09 (FR)**

(54) **Dérivés de l'indolo(3,2-c)quinoléine, leur procédé de préparation et leur activité antitumorale.**

(57) La présente invention est relative à de nouveaux dérivés de la 11-H indolo [3,2-c]quinoléine répondant à la formule suivante :

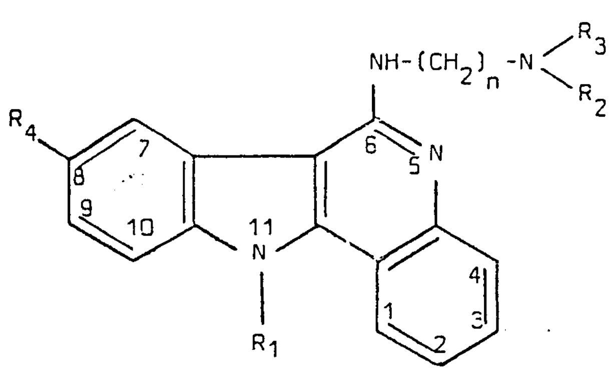

dans laquelle n représente un nombre entier de 2 à 4, $R_1$ représente l'hydrogène ou un groupe alcoyle inférieur, $R_2$ et $R_3$ représentent l'hydrogène ou un groupe alcoyle inférieur, ou bien $R_2$ et $R_3$ forment ensemble et avec l'atome d'azote auquel ils sont rattachés un hétérocycle saturé pouvant comporter un second hétéroatome tel que l'oxygène, le soufre ou l'azote, $R_4$ représente un hydroxyle OH ou un groupe alcoyloxy inférieur et leurs sels d'addition avec les acides minéraux ou organiques pharmaceutiquement acceptables ainsi que les formes tautomères lorsqu'elles existent.

L'invention est également relative à leur procédé de préparation, à leur application thérapeutique en tant qu'antitumoraux et à des intermédiaires de synthèse.

EP 0 226 508 A1

**Description**

Dérivés de l'indolo[3,2-c]quinoléine, leur procédé de préparation et leur activité antitumorale.

La présente invention est relative à de nouveaux dérivés de la 11-H indolo [3,2-c]quinoléine, à leur procédé de préparation, à leurs applications en thérapeutique et à des intermédiaires de synthèse.

Ces composés qui possèdent des propriétés antitumorales intéressantes répondent à la formule générale suivante :

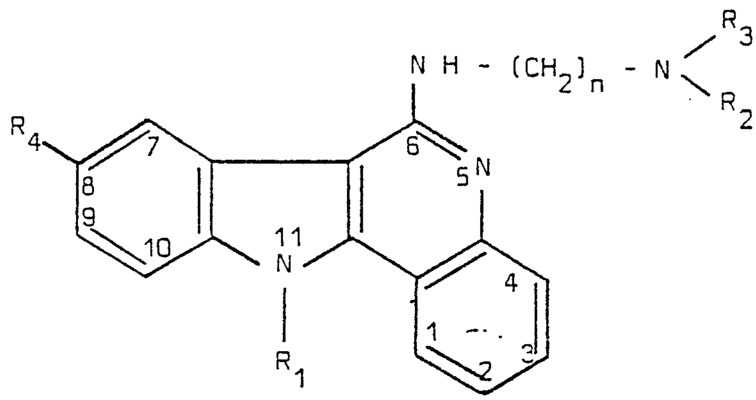

(I)

dans laquelle n représente un nombre entier de 2 à 4, $R_1$ représente l'hydrogène ou un groupe alcoyle inférieur, $R_2$ et $R_3$ identiques ou différents représentent l'hydrogène ou un groupe alcoyle inférieur ou bien $R_2$ et $R_3$ forment ensemble et avec l'atome d'azote auquel ils sont rattachés un hétérocycle saturé pouvant comporter un second hétéroatome tel que l'oxygène, le soufre ou l'azote, $R_4$ représente un hydroxyle OH ou un groupe alcoyloxy inférieur.

Par groupe alcoyle inférieur, on entend une chaîne hydrocarbonée saturée en $C_1$-$C_4$, linéaire ou ramifiée.

L'invention concerne aussi les formes tautomères de la formule I lorsqu'elles existent, ainsi que les sels d'addition avec les acides minéraux ou organiques pharmaceutiquement acceptables.

Quelques dérivés de la 11-H indolo[3,2-c]quino léine ont été décrits en tant qu'antimalariens. Il s'agit de dérivés alcoylaminoalcoylés en position -5 [W.O KERMACK, N.E STOREY ; J. Chem. Soc., 607-612 [1950)], de dérivés alcoylaminoalcoylés en position -11 [W.O KERMACK, N.E STOREY ; J. Chem. Soc., 607-612 (1950) et H. DEMARNE, Brevet Français No 2 327 783] et d'un dérivé diéthylaminométhylé en position -9 [V.E. MARQUEZ, J.W. CRANSTON, R.W. RUDDON, L.B. KIER, J.H. BURCKALTER, J. Med. Chem., 15 (I), 36-39 (1972)].

L'invention a également pour objet un procédé de préparation des composés de formule (I) précitée caractérisé en ce que :

a) par réaction de l'oxychlorure de phosphore (PO $Cl_3$) sur une 11-H N-oxyde indolo[3,2-c]quinoléine de formule II :

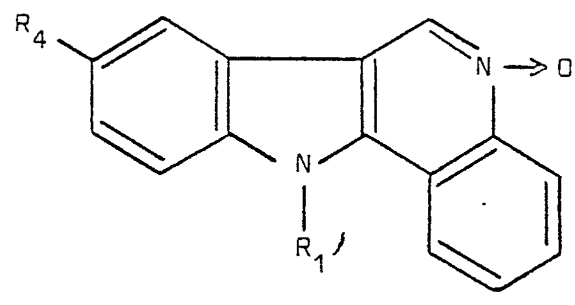

II

dans laquelle $R'_1$ représente un groupe alcoyle en $C_1$-$C_4$ ou le groupe tétrahydropyrannyle-2 et $R_4$ représente un groupe alcoyloxy en $C_1$-$C_4$, puis le cas échéant par action d'une solution aqueuse d'acide chlorhydrique sur les composés tétrahydropyrannylés ainsi obtenus afin de préparer les dérivés non substitués en position 11 ($R_1 = H$), on obtient les chloro-6 11-H indolo[3,2-c]quinoléines correspondantes de formule III, isolées sous forme de base ou de chlorhydrate :

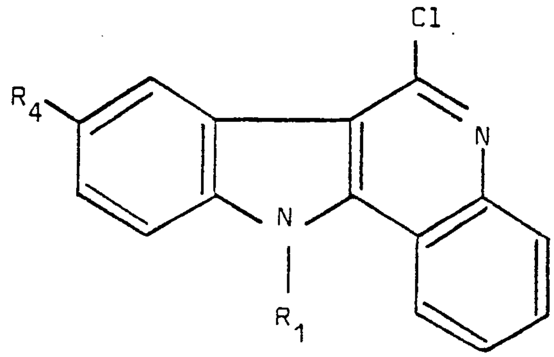

III

dans laquelle $R_1$ a la même signification que dans la formule I et $R_4$ représente un groupe alcoyloxy en $C_1$-$C_4$,

b) on condense le composé de formule III avec une amine de formule IV :

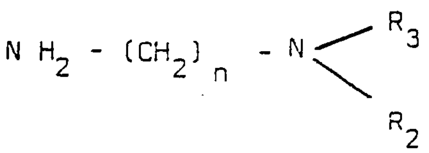

IV

dans laquelle n, $R_2$ et $R_3$ ont les significations précitées, pour obtenir le composé de formule I recherché dans laquelle $R_4$ représente un groupe alcoyloxy en $C_1$-$C_4$, et le cas échéant on prépare des composés de formule I dans lesquels $R_4$ représente un hydroxyle OH par désalcoylation des composés de formule I précédemment obtenus dans lesquels $R_4$ représente un radical alcoyloxy, à l'aide d'une solution aqueuse concentrée d'acide bromhydrique ou de tout autre agent de désalcoylation tels que l'acide iodhydrique, ou le chlorhydrate de pyridine à une température voisine de 100° C.

Les 11-H N-oxyde indolo[3,2-c]quinoléines (composés de formule II) sont préparées selon la méthode décrite par H. Demarne (brevet français No 2 327 783) à partir des (nitro-2 phényl)-2 indoles appropriées qui sont transformées en formyl-3 (nitro-2 phényl)-2 indoles par action de N,N diméthylformamide en présence d'oxychlorure de phosphore, puis en N-oxyde indolo[3,2-c]quinoléine par hydrogénation catalytique.

La formation des composés chlorés de formule III dans lesquels $R_1$ représente un radical alcoyle en $C_1$-$C_4$ ou le groupe tétrahydropyrannyl-2 s'effectue à une température comprise entre 10° C et la température de reflux de l'oxychlorure de phosphore.

La réaction de coupure du groupe tétrahydropyranyle qui conduit aux composés de formule III dans lesquels $R_1$ représent l'hydrogène s'effectue avantageusement par chauffage à 100° C.

La réaction d'amination des composés chlorés de formule III qui conduit aux composés de formule I, s'effectue, éventuellement dans un solvant inerte tel que l'éther diphénylique, à une température comprise entre 120° C et 200° C correspondant en général à la température de reflux de l'amine de formule IV utilisée.

Les exemples non limitatifs suivants sont donnés à titre d'illustration de l'invention.

## EXEMPLE 1

Préparation de la chloro-6 méthoxy-8 méthyl-11 11-H indolo [3,2-c]quinoléine (composé de formule III) ($R_1 = CH_3$, $R_4 = OCH_3$).

a) préparation du méthoxy-5 (nitro-2 phényl)-2 indole.

On introduit dans un réacteur de 250 ml, 18 g (0,13 mole) de paraméthoxyphénylhydrazine, 21,5 g (0,12 mole) de nitro-2 acétophénone, 160 ml d'éthanol absolu et 2,2 ml d'acide acétique. Le milieu réactionnel est agité sous atmosphère d'azote, à l'abri de la lumière, et à température ambiante pendant 20 heures. L'hydrazone obtenue précipite, elle est filtrée, lavée à l'éther isopropylique glacé et séchée. On isole ainsi 27,3 g de cristaux de couleur orange.

Rendement : 80 % ; F : 133° C.

Puis dans un réacteur d'un litre, on chauffe sous atmosphère d'azote 400 g d'éther éthylique de l'acide polyphosphorique (P.P.E.), à 60° C. On additionne ensuite par fraction 41 g de l'hydrazone préparée ci-dessus et le milieu réactionnel est porté à 60-65° C durant 5 heures sous agitation et à l'abri de la lumière. Après avoir laissé revenir à température ambiante on verse le mélange réactionnel dans 2 litres d'eau et de glace, on agite 15 minutes puis on extrait au dichlorométhane. On lave les phases organiques à l'eau, on sèche sur sulfate de magnésium et on concentre sous pression réduite.

L'huile brune obtenue est ensuite purifiée par passage sur colonne de silice (éluant : dichlorométhane). On obtient ainsi 25 g de cristaux jaunes de méthoxy-5 (nitro-2 phényl)-2 indole.

Rendement : 65 % ; F : 110°C.
Calc. pour $C_{15}H_{12}N_2O_3$ : C 67,15 ; H 4,51 ; N 10,44 %
trouvé : C 67,28 ; H 4,53 . N 10,37 %

b) Préparation du méthoxy-5 méthyl-1 (nitro-2 phényl)-2 indole.

A une suspension de 9,84 g d'hydrure de sodium à 50 % dans l'huile (0,205 mole) dispersée dans 100 ml de N,N-diméthylformamide, on ajoute goutte à goutte et sous courant d'azote une solution de 50 g (0,186 mole) de méthoxy-5 (nitro-2 phényl)-2 indole dans 300 ml de N,N-diméthylformamide. On chauffe le milieu à 35°C jusqu'à cessation du dégagement d'hydrogène puis le courant d'azote est coupé et 50 g (0,352 mole) d'iodure de méthyle sont ajoutés lentement. Après cette addition, le milieu est chauffé une heure à 50°C.

Le N,N-diméthylformamide est alors évaporé sous pression réduite. Le produit brut obtenu est repris par 100 ml d'eau, filtré et recristallisé dans le méthanol. On isole ainsi 49 g de méthoxy-5 méthyl-1 (nitro-2 phényl)-2 indole sous forme de cristaux orange.

Rendement: 93 % ; F : 144°C.
Calc. pour $C_{16}H_{14}N_2O_3$ : C 68,07% ; H 5,00% ; N 9,92 % ;
trouvé : C 68,16% ; H 4,86% ; N 9,99 % ;

c) Préparation du formyl-3 méthoxy-5 méthyl-1 (nitro-2 phényl)-2 indole.

Dans 200 ml de N,N-diméthylformamide refroidi à 0°C, on introduit progressivement 51,5 g (0,336 mole) d'oxychlorure de phosphore de façon à ce que la température du milieu ne dépasse pas 5°C. On ajoute ensuite 31,6 g (0,112 mole) de méthoxy-5 méthyl-1 (nitro-2 phényl)-2 indole dissous dans le minimum de N,N-diméthylformamide en maintenant la température du milieu réactionnel entre 5°C et 10°C. Puis on laisse la réaction se poursuivre pendant une heure à cette température. Le milieu réactionnel est alors versé dans 400 ml d'une solution aqueuse d'hydroxyde de sodium 5 N qui est ensuite chauffée pendant une heure à 60°C.

Le précipité formé puis est filtré, lavé à l'eau jusqu'à pH neutre puis à l'éther isopropylique. Après séchage, on obtient 27,8 g de poudre orangée.

Rendement : 80 % ; F : 162°C.
Calc. pour $C_{17}H_{14}N_2O_4$ : C 65,79 ; H 4,55 ; N 9,03
trouvé : C 65,59 ; H 4,55 ; N 9,04
IR (KBr) : ν c = 0 : 1660 $cm^{-1}$.

d) Préparation du N-oxyde de méthoxy-8 méthyl-11 11-H indolo [3,2-c] quinoléine (composé de formule II)

Dans un appareillage à hydrogénation sous pression normale préalablement purgé à l'azote, on introduit une suspension de 6 g de charbon palladié à 10 % dans 10 ml d'éthanol à 95 % puis une solution de 53,4 g (0,172 mole) de formyl-3 méthoxy-5 méthyl-1 (nitro-2 phényl)-2 indole dissous dans 500 ml de N,N-diméthylformamide. Puis sous bonne agitation et à température de 50°C, le réacteur est mis en communication avec le réservoir d'hydrogène dont 7,7 litres sont absorbés. La réaction terminée, le réacteur est purgé à l'azote puis le milieu est chauffé à 100°C. A cette température le catalyseur est filtré et le milieu est concentré sous pression réduite. Le produit brut est filtré puis recristallisé dans l'acide acétique. Après séchage à l'étuve sous vide, on isole ainsi 37 g de N-oxyde de méthoxy-8 méthyl-11 11-H indolo [3,2-c]quinoléine sous forme de poudre jaune solvatée avec de l'acide acétique.

Rendement : 60 % ; F : 260°C.
Calc. pour $C_{17}H_{14}N_2O_2$ ; 1,3 $C_2H_4O_2$ : C 66,05 ; H 5,39 ; N 7,86 %
Trouvé : C 65,90 ; H 5,21 ; N 8,18 %
IR (KBr) : absence de νc = 0 à 1660 $cm^{-1}$
présence de νc = 0 (-$CO_2H$) à : 1705 $cm^{-1}$

e) Préparation de la chloro-6 méthoxy-8 méthyl-11 11-H indolo [3,2-c] quinoléine (composé de formule III)

On porte au reflux sous courant d'azote et pendant trois heures une suspension de 1,1 g (0,00308 mole) de N-oxyde de méthoxy-8 méthyl-11 11-H indolo[3,2-c]quinoléine solvaté dans 40 ml d'oxychlorure de phosphore fraîchement distillé.

Après refroidissement du milieu réactionnel, le précipité obtenu est filtré et lavé plusieurs fois à l'éther, le précipité est alors mis en suspension dans 25 ml d'eau dans lesquels on ajoute ensuite 1 g de carbonate de sodium sous bonne agitation. Le milieu est ensuite extrait par du dichlorométhane. La phase organique est lavée à l'eau, séchée et évaporée à sec sous pression réduite. Le précipité obtenu est séché à 50°C sous vide. On isole ainsi 0,76 g de chloro-6 méthoxy-8 méthyl-11 11-H indolo[3,2-c]quinoléine sous forme de poudre blanche partiellement hydratée.

Rendement : 80 % ; F : 190°C.
Calc. pour $C_{17}H_{13}N_2ClO$ : 0,6 $H_2O$ : C 66,38 ; H 4,61 ; N : 9,10 %
Trouvé : C 66,45 ; H 4,23 ; N 8,86 %

EXEMPLE 2.

Préparation de la chloro-6 méthoxy-8 11-H indolo[3,2-c]quinoléine (composé de formule III) (R = H, $R_4 = OCH_3$).

a) Préparation du méthoxy-5 (nitro-2 phényl)-2 (tétrahydropyran-2 yl)-1 indole.

Dans 300 ml de N,N-diméthylformamide anhydre, on ajoute 100 g (0,37 moles) de méthoxy-5 (nitro-2 phényl)-2 indole. Puis, sous courant d'azote et à température ordinaire, on introduit progressivement 18 g (0,37 mole) d'hydrure de sodium à 50 % en suspension dans l'huile. Le milieu réactionnel est ensuite chauffé à 50° C pendant 15 minutes environ jusqu'à arrêt du dégagement d'hydrogène. Après refroidissement à 10° C, on ajoute goutte à goutte 68 g (0,5 mole) de chloro-2 tétrahydropyranne fraîchement distillé. On maintient sous agitation le milieu réactionnel à 20° C pendant 12 heures.

Le milieu réactionnel est alors versé dans 500 ml d'eau, puis extrait au dichlorométhane. La phase organique est lavée à l'eau, séchée, concentrée sous pression réduite. Le précipité obtenu est lavé à l'hexane puis à l'éther isopropylique. On isole ainsi 77 g de méthoxy-5 (nitro-2 phényl)-2 (tétrahydropyran-2 yl)-1 indole sous forme de poudre jaune qui est engagée dans l'étape suivante sans autre purification. F : 136° C.

b) Préparation du formyl-3 méthoxy-5 (nitro-2 phényl)-2 (tétrahydropyran-2 yl)-1 indole.

Dans 200 ml de N,N-diméthylformamide anhydre, on additionne lentement sous agitation 41 g (0,27 mole) d'oxychlorure de phosphore en maintenant la température du milieu réactionnel inférieure à 5° C. Puis on y introduit goutte à goutte la solution de 31,5 g de méthoxy-5 (nitro- 2 phényl)-2 (tétrahydropyran-2 yl)-1 indole dissous dans 200 ml de N,N-diméthylformamide sans que la température du milieu réactionnel ne dépasse 10° C. Enfin, on maintient la température du milieu à 20° C pendant une heure.

Le milieu réactionnel est alors versé dans 400 ml d'une solution aqueuse d'hydroxyde de sodium 5 N en refroidissant afin que la température n'excède pas 60° C. Puis le milieu est maintenu à 60° C pendant 30 minutes, puis il est refroidi et extrait au chlorure de méthylène.

La phase organique est lavée à l'eau, séchée et concentrée sous pression réduite. On isole ainsi 27 g de formyl-3 méthoxy-5 (nitro-2 phényl)-2 (tétrahydropyran-2 yl)-1 indole sous forme d'huile qui sont engagés dans l'étape suivante.

c) Préparation du N-oxyde de méthoxy-8 (tétrahydropyran-2 yl)-11 11-H indolo[3,2-c]quinoléine (composé de formule II ). ($R_1$ = tétrahydropyranyl-2, $R_4 = OCH_3$).

Dans un appareillage pour hydrogénation sous pression normale préalablement purgé à l'azote, on introduit une suspension de 3 g de platine sur charbon à 10 % dans 5 ml d'éthanol à 95 %. Puis on ajoute 27 g de formyl-3 méthoxy-5 (nitro-2 phényl)-2 (tétrahydropyran-2 yl)-1 indole dissous dans 200 ml de N,N-diméthylformamide.

Sous agitation et à température ordinaire, le réacteur est alors mis en communication avec le réservoir d'hydrogène dont 400 ml sont absorbés. Le réacteur est ensuite purgé à l'azote puis, après filtration du catalyseur, la phase organique est concentrée sous pression réduite. Le produit brut obtenu est repris par le cyclohexane, filtré et recristallisé dans l'isopropanol.

On obtient ainsi 21,1 g de N-oxyde de méthoxy-8 (tétrahydropyran-2 yl)-11 11-H indolo [3,2-c]quinoléine, (rendement de 40 % par rapport au (nitro-2 phényl)-2 méthoxy-5 indole). F : 240° C.
Calc. pour $C_{21}H_{20}N_2O_3$ : C 72,39% H 5,78%; N 8,04 %
Trouvé : C 72,33%; H 5,74%; N 7,93 %

d) Préparation de la chloro-6 méthoxy-8 (tétrahydropyran-2 yl)-11 11-H indolo[3,2-c]quinoléine.

Sous atmosphère d'azote, on ajoute progressivement 18 g (0,05 mole) de N-oxyde de méthoxy-8 (tétrahydropyran-2 yl)-11 11-H indolo[3,2-c]quinoléine dans 250 ml d'oxychlorure de phosphore ($POCL_3$) fraîchement distillé. La température du milieu réactionnel s'élève à 35° C. Cette température est maintenue pendant 15 minutes et sous bonne agitation. La solution est homogène en début de réaction, puis après 10 minutes, le chlorhydrate du dérivé chloré formé précipite. Après réaction, il est filtré et lavé à l'éther éthylique. Le précipité est alors ajouté à une solution aqueuse d'hydrogénocarbonate de sodium (NaH $CO_3$) ; la base ainsi libérée est extraite au dichlorométhane. La phase organique est lavée à l'eau, séchée et concentrée sous pression réduite.

Le produit brut est purifié sur colonne de silice (éluant : $CH_2Cl_2$).

On obtient 12,8 g de chloro-6 méthoxy-8 (tétrahydropyran-2 yl)-11 11-H indolo[3,2-c]quinoléine pure.
Rendement : 67,5 % ; F 140° C
Calc. pour $C_{21}H_{19}N_2O_2Cl$ : C 68,75 ; H 5,22 ; N 7,64 %
Trouvé : C 68,47 : H 5,14 ; N 7,54 %

e) Préparation du chlorhydrate de chloro-6 méthoxy-8 11-H indolo[3,2-c]quinoléine (composé de formule III) $R_1$ = H, $R_4 = OCH_3$).

Une suspension de 40 g de chloro-6 méthoxy-8 (tétrahydropyran-2 yl)-11 11-H indolo[3,2-c]quinoléine dans 500 ml d'une solution aqueuse d'acide chlorhydrique 4 N est portée au reflux pendant une heure. Après refroidissement, la solution est filtrée. Le précipité est lavé à l'acétone puis à l'éther éthylique. On obtient ainsi 30 g de chlorhydrate de chloro-6 méthoxy-8 11-H indolo[3,2-c]quinoléine.
Rendement : 96,9 % ; F : 220° C.

Calc. pour $C_{16}H_{12}N_2O\ Cl_2$ : C 60,20 ; H 3,79 ; N 8,78 %
Trouvé : C 59,94 ; H 3,83 ; N 8,72 %

EXEMPLE 3.

Préparation de la (diéthylamino-3 propylamino)-6 méthoxy-8 méthyl-11 11-H indolo[3,2-c]quinoléine, et de son dichlorhydrate (SR 25439 A : dérivé N° 1).

La chloro-6 méthoxy-8 méthyl-11 11-H indolo[3,2-c]quinoléine de formule III ($R_1 = CH_3$ ; $R_4 = OCH_3$) (3,2 g) est chauffée sous atmosphère d'azote dans la diéthylamino-3 propylamine (29 g) à 170-180°C pendant 3 H 30 en suivant la disparition du dérivé chloré par chromatographie sur couche mince (silice ; éluant : chlorure de méthylène-méthanol 9/1). Puis l'excès d'amine est éliminé sous pression réduite. Le résidu est repris par 100 ml d'une solution aqueuse d'hydroxyde de sodium N et extrait au chlorure de méthylène. La phase organique est séchée puis concentrée sous pression réduite. Le produit brut est lavé au cyclohexane et recristallisé dans l'éther isopropylique.
Rendement : 70 % ; F : 92°C.
Calc. pour $C_{24}H_{30}N_4O$ : C 73,81 ; H 7,74 ; N 14,35 %
Trouvé : C 73,57 ; H 7,84 ; N 14,48 %

On solubilise, à chaud dans le méthanol, 2 g de la base précédemment obtenue. Puis on y ajoute progressivement une solution anhydre d'acide chlorhydrique dans l'éther éthylique. Après refroidissement, le dichlorhydrate précipite. Il est filtré, lavé à l'éther éthylique et séché.
Rendement : 92 % ; F : 236°C.
Calc. pour $C_{24}H_{32}N_4O\ Cl_2$ ; $2H_2O$ : C 57,71 ; H 7,26 ; N 11,22 %
Trouvé : C 57,83 ; H 7,21 ; N 11,00
RMN $H_1$ [$(CD_3)_2SO$] ; δ 1,25 (t, 2 x 3H, $CH_2$-$\underline{CH_3}$) ; 2,30 (m, 2H, $CH_2$-β) ; 2,8-3,5 (m, 3 x 2H, $\underline{CH_2}$-$CH_3$ + $CH_2$-γ) ; 3,6-4,4 (m, 8H, N-$CH_3$ + $CH_2$-α + O-$\underline{CH_3}$) ; 6,9-8 (m, 7H, protons aromatiques).

EXEMPLE 4.

Préparation de la (diméthylamino-3 propylamino)-6 méthoxy-8 méthyl-11 11-H indolo[3,2-c]quinoléine, et de son dichlorhydrate (SR 25641 A : dérivé n° 2).

Ce composé est préparé selon le même mode opératoire que dans l'exemple 3 à partir de la chloro-6 méthoxy-8 méthyl-11 11-H indolo[3,2-c]quinoléine de formule III ($R_1 = CH_3$ ; $R_4 = OCH_3$) (4,9 g) et de la diméthylamino-3 propylamine (50 ml), la réaction étant réalisée à 130°C pendant 6 heures. La recristallisation de la base est effectuée dans l'acétate d'éthyle.
Rendement : 70 % ; F : 260°C.
Calc. pour $C_{22}H_{26}N_4O$ : C 72,89 ; H 7,23 ; N 15,45 %
Trouvé : C 72,70 ; H 7,28 ; N 15,62 %
RMN $H_1$ [$(CD_3)_2SO$] ; δ 2 (m, 2H, $CH_2$-β) ; 2,1-2,7 (m, 8H, $N(CH_3)_2$ + $CH_2$-γ) ; 3,4-4,1 (m, 5H, $CH_2$-α + N-$CH_3$) ; 4,2 5 s 3H, O-$CH_3$) ; 6,8-8 (m, 7H, protons aromatiques) ; (en présence de $D_2O$).

Le dichlorhydrate est obtenu de façon identique à celle de l'exemple 3 avec un rendement de 93 % ; F : 260°C.
Calc. pour $C_{22}H_{28}N_4O\ Cl_2$ ; 0,85 $H_2O$ : C 58,51 ; H 6,58 ; N 12,41 %
Trouvé : C 58,24 ; H 6,19 ; N 12,72 %

EXEMPLE 5.

Préparation de la (diéthylamino-3 propylamino)-6 hydroxy-8 méthyl)-11 11-H indolo[3,2-c]quinoléine, et de son dichlorhydrate.

[SR 25440 A : dérivé N° 3].

Une solution de (diéthylamino-3 propylamino)-6 méthoxy-8 méthyl-11 11-H indolo [3,2-c]quinoléine (5,8 g) dans 100 ml d'une solution aqueuse d'acide bromhydrique à 48 % est portée au reflux pendant 4 heures. Après refroidissement, on y additionne progressivement une solution d'ammoniaque jusqu'à pH basique. Le précipité obtenu est dissous dans le méthanol, la solution méthanolique est séchée puis concentrée sous pression réduite.

La base ainsi obtenue est solubilisée dans le méthanol auquel on ajoute progressivement une solution anhydre d'acide chlorhydrique dans l'éther éthylique. Le dichlorhydrate précipite, il est filtré, lavé à l'éther et enfin recristallisé dans l'éthanol à 95 %.
Rendement : 40 % ; F > 260°C.
Calc. pour $C_{23}H_{30}N_4O\ Cl_2$ ; 0,5 $H_2O$ : C 60,26 ; H 6,82 ; N 12,22 %
Trouvé : C 60,15 ; H 6,89 ; N 12,20 %
RMN $H_1$ ($D_2O$) ; δ 1,35 (t, 2 x 3H, $CH_2$-$\underline{CH_3}$) ; 2,50 (m, 2H, $CH_2$-β) ; 2,8-3,6 (m, 11H, $\underline{CH_2}$-$CH_3$ + $CH_2$-α + $CH_2$-γ + N-$CH_3$) ; 7,1-8 (m, 7H, protons aromatiques).

EXEMPLE 6.

Préparation de la (diméthylamino-3 propylamino)-6 hydroxy-8 méthyl-11 11-H indolo[3,2-c]quinoléine, et de son dichlorhydrate [SR 25648 A : dérivé No4].

Une solution de (diméthylamino-3 propylamino)-6 méthoxy-8 méthyl-11 11-H indolo[3,2-c]quinoléine (2,6 g) dans 50 ml d'une solution aqueuse d'acide bromhydrique à 48 % est portée au reflux pendant 4 heures. Après refroidissement, on y additionne progressivement une solution d'ammoniaque jusqu'à pH basique. Le précipité obtenu est dissout dans le méthanol. La solution méthanolique est séchée puis concentrée sous pression réduite. La base ainsi obtenu est recristallisée dans le méthanol.

Rendement : 72 % ; F > 260°C.

Calc. pour $C_{21}H_{24}N_4O$ : C 72,38 ; H 6,94 ; N 16, 08 %

Trouvé : C 72,48 ; H 7,02 ; N 16,12 %

RMN $H_1$ [$(CD_3)_2$ SO] ; δ 2,1 (m, 2H, $CH_2$-β) ; 2,2-2,8 (m, 8H, $N(CH_3)_2$ + $CH_2$-γ) ; 3,7-4,5 (m, 5H, N-$CH_3$ + $CH_2$-α) ; 6,9-8 (m, 7H, protons aromatiques) ; (en présence de $D_2O$).

Le dichlorhydrate est préparé par dissolution de la base dans le méthanol et addition d'une solution anhydre d'acide chlorhydrique dans l'éther éthylique. Après refroidissement il est filtré, lavé à l'éther éthylique et séché.

Rendement : 98 % ; F > 260°C.

Calc. pour $C_{21}H_{26}N_4O$ $Cl_2$ ; 0,8 $H_2O$ : C 57,83 ; H 6,33 ; N 12,85 %

Trouvé : C 57,84 ; H 6,19 ; N 12,88 %

EXEMPLE 7.

Préparation de la (diéthylamino-3 propylamino)-6 méthoxy-8 11-H indolo[3,2-c]quinoléine, et de son dichlorhydrate. [SR 25525 A : dérivé No 5].

La chloro-6 méthoxy-8 11-H indolo[3,2-c]quinoléine de formule III ($R_1$ = -H ; $R_4$ = -O $CH_3$) (14 g) est chauffée sous atmosphère d'azote dans 100 ml de diéthylamino-3 propylamine à 170°C pendant 3 H 30, en suivant la disparition du dérivé chloré par chromatographie sur couche mince (silice ; éluant : chlorure de méthylèneméthanol 9/1). Puis l'excès d'amine est éliminé sous pression réduite. Le résidu est repris par 300 ml d'une solution aqueuse d'hydroxyde de sodium et extrait au chlorure de méthylène. La phase organique est lavée à l'eau, séchée et concentrée sous pression réduite. La base ainsi obtenu est solubilisée dans le méthanol, puis on y ajoute progressivement une solution anhydre d'acide chlorhydrique dans l'éther éthylique. Après refroidissement, le dichlorhydrate précipite. Il est filtré et recristallisé dans l'éthanol à 95 %.

Rendement : 84 % ; F > 260°C.

Calc. pour $C_{23}H_{30}N_4O$ $Cl_2$ ; $3H_2O$ : C 54,87 ; H 7,21 ; N 11,13 %

Trouvé : C 54,69 ; H 7,38 ; N 11,30 %

RMN $H_1$ ($D_2O$) ; δ 1,45 (t, 2 x 3H, $CH_2$-$\underline{CH_3}$) ; 2,25 (m, 2H, $CH_2$-β) ; 2,7-3,6 (m, 11H, $\underline{CH_2}$-$CH_3$ + $CH_2$-α + $CH_2$-γ + $OCH_3$) ; 7,05-8 (m, 7H, protons aromatiques).

EXEMPLE 8.

Préparation de la (diméthylamino-3 propylamino)-6 méthoxy-8 11-H indolo [3,2-c]quinoléine, et de son dichlorhydrate (SR 25554 A : dérivé No 6].

Ce composé est préparé selon le même mode opératoire que dans l'exemple 7 à partir de la chloro-6 méthoxy-8 11-H indolo[3,2-c]quinoléine de formule III ($R_1$ =-H, $R_4$ = -$OCH_3$) (3,55 g) et de diméthylamino-3 propylamine (50 ml), la réaction étant réalisée à 130°C pendant 4 heures.

La recristallisation du dichlorhydrate est effectuée dans le méthanol.

Rendement : 45 % ; F > 260°C.

Calc. pour $C_{21}H_{26}N_4O$ $Cl_2$ ; 2,75 $H_2O$ : C 53,57 ; H 6,74 ; N 11,90

Trouvé : C 53,57 ; H 6,55 ; N 12,25

RMN $H_1$ ($0_2O$) ; δ 2,2 (m, 2H, $CH_2$-β) ; 3,1 (s, 2 x 3H, $N(CH_3)_2$ ; 3,1-3,8 (m, 4H, $CH_2$-α + $CH_2$-γ) ; 3,8 (s, 3H, $OCH_3$ ; 6,5-7,9 (m, 7H, protons aromatiques).

EXEMPLE 9.

Préparation de la (diéthylamino-2 éthylamino)-6 méthoxy-8 11-H indolo[3,2-c]quinoléine, et de son dichlorhydrate [SR 25743 A : dérivé No 7].

Ce composé est préparé selon le mode opératoire de l'exemple 7 à partir du chlorhydrate de chloro-6 méthoxy-8 11-H indolo[3,2-c]quinoléine de formule III ($R_1$ =-H, $R_4$ = $OCH_3$) (5 g) et de la diéthylamino-2 éthylamine (50 ml) portée au reflux pendant 3 heures. La base obtenue est re cristallisée dans le méthanol (rendement : 84 %). Puis elle est dissoute dans l'éther éthylique auquel on ajoute progressivement une solution anhydre d'acide chlorhydrique dans l'éther éthylique. Le dichlorhydrate précipite, il est filtré et séché.

Rendement : 80 % ; F > 260°C.

Calc. pour $C_{22}H_{28}N_4O$ $Cl_2$ ; 2,75 $H_2O$ : C 54,48 ; H 6,96 ; N 11,55 ; Cl 14,62 %

Trouvé : C 54,48 ; H 7,04 ; N 11,89 ; Cl 14,62.%

RMN $H_1$ ($0_2O$) ; δ 1,30 (t, 2 x 3H, $CH_2$-$\underline{CH_3}$) ; 3,30 (m, 2 x 2H, $\underline{CH_2}$-$CH_3$) 3,5 (m, 2H, $CH_2$-α) ; 3,8-4 (m, 5H),

$CH_2$-β) ; 6,20-7,5 (m, 7H, protons aromatiques).

EXEMPLE 10.

Préparation de la méthoxy-8 (morpholino-3 propylamino)-6 11-H indolo[3,2-c]quinoléine, et de son dichlorhydrate [SR 25745 A : dérivé No 8].

On dissout à chaud 0,0125 mole de chlorhydrate de chloro-6 méthoxy-8 11-H indolo[3,2-c]quinoléine (4 g) dans 40 ml de diphényl éther auxquels on ajoute 0,0376 mole de morpholino-3 propylamine (5,5 g) et 0,0275 mole de carbonate de potassium (4 g). Le milieu réactionnel est chauffé à 180°C pendant 3 heures. Après refroidissement il est filtré. On ajoute alors à la phase organique une solution anhydrde d'acide chlorhydrique dans l'éther éthylique (6 N). Le dichlorhydrate précipite, il est filtré et lavé à l'éther éthylique avant d'être recristallisé dans l'eau. Après séchage, on isole 4,3 g du dichlorhydrate.

Rendement : 75 % ; F > 260°C.

Calc. pour $C_{23}H_{28}N_4O_2$ $Cl_2$ ; 1,6 $H_2O$ : C 56,10 ; H 6,39 ; N 11,38 %

Trouvé : C 56,09 ; H 6,79 ; N 11,33 %

RMN $H_1$ [$(CD_3)_2$ SO] ; δ 2,35 (m, 2H, $CH_2$-β) ; 3-3,7 (m, 3 x 2 H, $CH_2N$ cycle + $CH_2$-γ) ; 3,8-4,5 (m, 9H, $CH_2$-α + O-$CH_3$ + $CH_2$-O cycle) ; 7,1-8 (m, 7H, protons aromatiques) ; (en présence de $D_2O$).

EXEMPLE 11.

Préparation de la méthoxy-8 (pipéridino-3 propylamino)-6 11-H indolo [3,2-c]quinoléine, et de son dichlorhydrate [SR 25746 A : dérivé No 9].

Ce composé est préparé selon le même mode opératoire que l'exemple 10 à partir du chlorhydrate de chloro-6 méthoxy-8 11-H indolo [3,2-c]quinoléine et de pipéridino-3 propylamine

Rendement : 70 % ; F > 260°C.

Calc. pour $C_{24}H_{30}N_4O$ $Cl_2$ ; $2H_2O$ : C 57,94 ; H 6,83 ; N 11,26 %

Trouvé : C 57,67 ; H 6,74 ; N 11,47 %

RMN $H_1$ [$(CD_3)_2SO$] ; δ 1,85 (m, 3 x 2H $(CH_2)_3$ cycle) ; 2,30 (m, 2H, $CH_2$-β) ; 2,8-3,7 (m, 3 x 2H, $CH_2$-N cycle + $CH_2$-γ) ; 4 (m, 2H, $CH_2$-α) ; 4,1 (s, 3H, $OCH_3$) ; 7,2-8 (m, 7H, protons aromatiques) ; (en présence de $D_2O$).

EXEMPLE 12.

Préparation de l'(éthylamino-3 propylamino)-6 méthoxy-8 11-H indolo [3,2-c]quinoléine, et de son dichlorhydrate [SR 25769 A : dérivé No 10].

Ce composé est préparé selon le même mode opératoire que l'exemple 9 à partir du chlorhydrate de chloro-6 méthoxy-8 1-H indolo[3,2-c]quinoléine et d'éthylamino-3 propylamine. La base obtenue est recristallisée dans l'isopropanol (rendement : 61°C ; F = 180°C). Le dichlorhydrate est obtenu avec un rendement de 86 % ; F > 260°C.

Calc. pour $C_{21}H_{26}N_4O$ $Cl_2$ ; $2H_2O$ : C 57,40 ; H 6,42 ; N 12,75 ; Cl 16,14 %

Trouvé : C 57,77 ; H 6,36 ; N 12,53 ; Cl 16,05 %

RMN $H_1$ ($D_2O$) ; δ 1,4 (t, 3H, $CH_2$-$\underline{CH_3}$) ; 2,2 (m, 2H, $CH_2$-β) ; 3,2-3,4 (m, 2 x 2H, $\underline{CH_2}$-$CH_3$ + $CH_2$-γ) ; 3,60 (m, 2H, $CH_2$-α) ; 3,85 (s, 3H, $OCH_3$) ; 6,5-7,6 (m, 7H, protons aromatiques).

EXEMPLE 13.

Préparation de (diéthylamino-3 propylamino)-6 hydroxy-8 11-H indolo[3,2-c]quinoléine, dichlorhydrate [SR 25527 A : dérivé No 11].

Une solution de (diéthylamino-3 propylamino)-6 méthoxy-8 11-H indolo[3,2-c]quinoléine (9 g dans 150 ml d'une solution aqueuse d'acide bromhydrique à 48 % est portée au reflux pendant 3 heures. Après refroidissement, on y additionne progressivement une solution d'ammoniaque jusqu'à pH basique. Le précipité obtenu est dissous dans l'acétone, la solution organique est séchée et concentrée sous pression réduite.

La base ainsi obtenu est solubilisée dans le méthanol auquel on ajoute progressivement une solution anhydre d'acide chlorhydrique dans l'éther éthylique. Le dichlorhydrate précipite, il est filtré, lavé à l'éther éthylique et séché.

Rendement : 74 % ; F > 260°C.

Calc. pour $C_{22}H_{28}N_4O$ $Cl_2$ ; $2H_2O$ : C 56,05 ; H 6,84 ; N 11,83 %

Trouvé : C 56,24 ; H 6,63 ; N 11,67 %

RMN $H_1$ ($D_2O$) ; δ 1,4 (t, 2 x 3H, $CH_2$-$\underline{CH_3}$) ; 2,6-3,6 (m, 5 x 2H, $CH_2$-β) + $\underline{CH_2}$-$CH_3$ + $CH_2$-α + $CH_2$-γ) ; 7,1-8 (m, 7H, protons aromatiques).

EXEMPLE 14.

Préparation de la (diméthylamino-3 propylamino)-6 hydroxy-8 11-H indolo [3,2-c]quinoléine, et de son dichlorhydrate, [SR 25724 A : dérivé No 12].

Une solution de (diméthylamino-3 propylamino)-6 méthoxy-8 11-H indolo[3,2-c]quinoléine (1,5 g) dans 30 ml d'une solution aqueuse d'acide bromhydrique à 48 % est portée au reflux pendant 6 heures. Après refroidissement, on y ajoute une solution d'ammoniaque jusqu'à pH basique. Le précipité est dissous dans le chlorure de méthylène, la solution organique est lavée à l'eau, séchée et concentrée sous pression réduite. Le produit brut est alors purifié par chromatographie sur colonne de silice (éluant : méthanol-triéthylamine 9/1) on obtient ainsi la base purifiée (1 g, rendement : 66 %) qui est dissoute dans l'éthanol auquel on ajoute une solution anhydre d'acide chlorhydrique dans l'éther éthylique. Le dichlorhydrate précipite, il est filtré et recristallisé dans le méthanol.

Rendement : 60 % ; F > 260°C.

Calc. pour $C_{20}H_{24}N_4O$ $Cl_2$ ; 2,5 $H_2O$ : C 53,10 ; H 6,46 ; N 12, 38 ; Cl 15,67 %

Trouvé : C 53,10 ; H 5,70 ; N 12,26 ; Cl 15,53 %

RMN $H_1$ [$(CD_3)_2$ SO] ; δ 2,25 (m, 2H, $CH_2$-β) ; 2,95 (s, 2 x 3H, $N(CH_3)_2$) ; 3,1-3,6 (m, 2 x 2H, $CH_2$-α + $CH_2$-γ) 6,9-8 (m, 7H, protons aromatiques) (en présence de $D_2O$).

EXEMPLE 15.

Préparation de la (diéthylamino-2 éthylamino)-6 hydroxy-8 11-H indolo[3,2-c]quinoléine, et de son dichlorhydrate [SR 25757 A : dérivé No 13].

Une solution de (diéthylamino-2 éthylamino)-6 méthoxy-8 11-H indolo[3,2-c]quinoléine (3 g) dans 30 ml d'une solution aqueuse d'acide bromhydrique à 48 % est portée au reflux pendant 5 heures. Après refroidissement, on y ajoute une solution d'ammoniaque jusqu'à pH basique. Le précipité obtenu est repris par du méthanol auquel on ajoute une solution anhydre d'acide chlorhydrique dans l'éther éthylique. Le milieu réactionnel est porté au reflux jusqu'à l'obtention d'une solution homogène. Après refroidissement, le dichlorhydrate précipite. Il est filtré, lavé à l'éther éthylique et séché.

Rendement : 71 % ; F : 260°C.

Calc. pour $C_{21}H_{26}N_4O$ $Cl_2$ ; $H_2O$ : C 57,40 ; H 6,42 ; N 12,75 ; Cl 16,14 %

Trouvé : C 57,43 ; H 6,13 ; N 12,88 ; Cl 16,29 %

RMN $H_1$ ($D_2O$) ; δ 1,40 (t, 2 x 3H, $CH_2$-$\underline{CH_3}$) ; 3,3-3,55 (m, 3 x 2H, $\underline{CH_2}$-$CH_3$ + $CH_2$-α) ; 3,8 (m, 2H, $CH_2$-β) ; 6,9-7,9 (m, 7H, protons aromatiques).

EXEMPLE 16.

Préparation de l'(éthylamino-3 propylamino)-6 hydroxy-8 11-H indolo[3,2-c]quinoléine, et de son dichlorhydrate [SR 25758 A : dérivé No 14].

Une solution d'(éthylamino-3 propylamino)-6 méthoxy-8 11-H indoloquinoléine (2,4 g) dans 30 ml d'une solution aqueuse d'acide bromhydrique à 48 % est portée au reflux pendant 4 heures. Après refroidissement, le bromhydrate précipite, il est filtré, lavé à l'acétone et purifié sur colonne de silice (éluant : méthanol-triéthylamine 9/1) sous forme de base. La base ainsi obtenue est dissoute dans le méthanol auquel on ajoute une solution anhydre d'acide chlorhydrique dans l'éther éthylique. Le dichlorhydrate précipite, il est filtré, lavé à l'éther éthylique et séché.

Rendement : 57 % ; F > 260°C.

Calc. pour $C_{21}H_{24}N_4O$ $Cl_2$ ; 1,5 $H_2O$ : C 55,29 ; H 6,26 ; N 12,89 ; Cl 16,32 %

Trouvé : C 55,29 ; H 6,22 ; N 12,93 ; Cl 16,12 %

RMN $H_1$ ($D_2O$) ; δ 1,45 (t, 3H, $CH_2$-$\underline{CH_3}$) ; 2.15 (m, 2H, $CH_2$-β) ; 3,2-3,35 (m, 2 x 2H, $\underline{CH_2}$-$CH_3$ + $CH_2$-γ) ; 6,6-7,8 (m, 7H, protons aromatiques).

EXEMPLE 17.

Préparation d'hydroxy-8 (morpholino-3 propylamino)-6 11-H indolo[3,2-c]quinoléine, et de son dichlorhydrate [SR 25767 A : dérivé No 15].

Ce composé est préparé selon le même mode opératoire que l'exemple 13 à partir de méthoxy-8 (morpholino-3 propylamino)-6 11-H indolo[3,2-c]quinoléine sous forme de dichlorhydrate. Le dichlorhydrate est obtenu avec un rendement de 75 % ; F > 260°C.

Calc. pour $C_{22}H_{26}N_4O_2$ $Cl_2$ ; $2H_2O$ : C 54,43 ; H 5,77 ; N 11,54 %

Trouvé : C 54,48 ; H 6,14 ; N 11,31 %

RMN $H_1$ [$(CD_3)_2$ SO] ; δ 2,35 (m, 2H, $CH_2$-β) ; 3,37 (m, 3 x 2H, $CH_2$-N cycle + $CH_2$-γ) ; 3,8-4,4 (m, 3 x 2H, $CH_2$-α + $CH_2$-O cycle) ; 7,1-8,2 (m, 7H, protons aromatiques) ; (en présence de $D_2O$).

EXEMPLE 18.

Préparation d'hydroxy-8 (pipéridino-3 propylamino)-6 11-H indolo[3,2-c]quinoléine, et de son dichlorhydrate [SR 25768 A : dérivé No 16].

Une suspension de méthoxy-8 (pipéridino-3 propylamino)-6 11-H indolo[3,2-c]quinoléine sous forme de

dichlorhydrate (2,8 g) dans 50 ml d'une solution aqueuse d'acide bromhydrique à 48 % est portée au reflux pendant 3 heures. Après refroidissement, le dibromhydrate précipite, il est filtré et lavé à l'acétone. La base est régénérée par une solution d'ammoniaque et le dichlorhydrate est obtenu selon le mode opératoire de l'exemple 13.

Rendement : 70 % ; F > 260°C.

Calc. pour $C_{23}H_{28}N_4O\ Cl_2$ ;1,8 $H_2O$ : C 57,57 ; H 6,58 ; N 11,67 %

Trouvé : C 57,73 ; H 6,43 ; N 11,40 %

RMN $H_1$ [$(CD_3)_2SO$] ; δ 1,85 (m, 3 x 2H, $(CH_2)_3$ cycle) ; 2,3 (m, 2H, $CH_2$-β) ; 2,9-3,7 (m, 3 x 2H, $CH_2$-N cyle + $CH_2$-γ) ; 4,2 (m, 2H, $CH_2$-α) ; 7,1-8,2 (m, 7H, protons aromatiques) ; (en présence de $D_2O$).

Les résultats des études pharmacologiques et toxicologiques rapportées ci-après ont mis en évidence les intéressantes propriétés des dérivés de l'invention, tant sur le plan de la tolérance que sur le plan de leur activité antitumorale.

L'invention a donc encore pour objet un médicament présentant, en particulier, une activitié antitumorale, ca ractérisé en ce qu'il contient à titre de principe actif un dérivé de formule I ou l'une de ses formes tautomères lorsqu'elles existent ou un de ses sels d'addition avec les acides minéraux ou organiques pharmaceutiquement acceptables.

ETUDE TOXICOLOGIQUE.

Les composés de l'invention bénéficient d'une bonne tolérance et d'une faible toxicité.

Ainsi l'administration à des souris consanguines $DBA_2$ saines, en une injection unique par voie intrapéritonéale, d'une dose de 50 mg/kg des dérivés No 1 (SR 25439 A), No 3 (SR 25440 A), No 5 (SR 25525 A), No 11 (SR 25527 A), No 12 (SR 25724 A), No 14 (SR 25758 A), No 15 (SR 25767 A) et No 16 (SR 25768 A) n'a provoqué aucune mortalité évaluée au septième jour après l'administration.

La toxicité a été aussi évaluée en suivant, tout au long des essais, la mortalité et l'évolution pondérale des animaux d'expérience. On a constaté ainsi que cela est rapporté plus loin, qu'aux doses efficaces, les composés de l'invention étaient bien tolérés et que l'indice thérapeutique était nettement favorable.

ETUDE PHARMACOLOGIQUE.

Elle a été réalisée in vitro et in vivo.

1) In vitro : (PAOLETTI et al., Chem. Biol. Interaction, 1979, 25, 45-58).

L'activité cytotoxique des composés de l'invention a été évaluée par des essais in vitro. Ces essais consistent à ajouter des concentrations croissantes du composé à tester à une culture cellulaire de la lignée tumorale leucémie L 1210 en phase exponentielle de croissance. Les cellules sont incubées à 37°C dans une étuve à $CO_2$ et comptées toutes les 24 heures. Le calcul de l'$ID_{50}$ (concentration du produit exprimée en micromole $1^{-1}$ qui inhibe la prolifération cellulaire à 50 p. 100) est effectuée après 48 heures de contact. Ainsi déterminées les $ID_{50}$ des composés de l'invention sont rassemblées dans le tableau ci-après.

TABLEAU I

| DERIVE N° | N° SR | ID 50 ($\mu M\ l^{-1}$) |
|---|---|---|
| 1 | 25439 A | 0,35 |
| 2 | 25641 A | 0,40 |
| 3 | 25440 A | 0,06 |
| 4 | 25648 A | 0,04 |
| 5 | 25525 A | 0,69 |
| 6 | 25554 A | 0,80 |
| 7 | 25743 A | 2,1 |
| 8 | 25745 A | 0,61 |
| 9 | 25746 A | 0,64 |
| 10 | 25769 A | 0,58 |
| 11 | 25527 A | 0,13 |
| 12 | 25724 A | 0,08 |
| 13 | 25757 A | 2,6 |
| 14 | 25758 A | 1,4 |
| 15 | 25767 A | 0,28 |
| 16 | 25768 A | 0,18 |

In vivo : (GERAN et al., Cancer Chemother., 1972, 2, 07-57).

L'activité antitumorale a été testée in vivo sur différentes tumeurs selon le protocole général suivant:

Toutes les souris mâles ou femelles sont inoculées par voie intrapéritonéale au jour Jo avec une quantité déterminée de cellules tumorales viables, variable selon la tumeur.

Elles sont ensuite réparties en différents lots. Chaque lot correspondant aux animaux traités par le produit à tester à une dose déterminée est composé de 10 souris. Dans ce cas, le produit à tester est dissous dans l'eau distillée et il est administré, pendant n jours ($J_1$-$J_n$) variables selon le protocole choisi, par voie intrapéritonéale à raison de 0,1 ml par 10 g de poids des souris traitées, les doses du produit administré étant précisées dans les tableaux ci-dessous.

Le lot "contrôle négatif", correspondant aux animaux qui ne sont pas traités par un produit, comprend 2 $\sqrt{N}$ animaux, N étant égal au nombre total de souris traitées par un produit.

Enfin, tous les essais sont conduits parallèlement à l'étude d'un lot appelé "contrôle positif" de 10 animaux qui est traité par une molécule active, variable selon le protocole choisi, dans les mêmes conditions que les lots traités par le produit à tester, afin de valider l'essai.

L'activité antitumorale est évaluée en considérant l'augmentation du temps de survie des animaux traités par rapport à celui des animaux du lot "contrôle négatif" selon la formule :

$$T/C = \frac{\text{Jour médian de survie des animaux traités à une dose donnée de produit}}{\text{Jour médian de survie des animaux "contrôle négatif"}} \times 100$$

Un composé est considéré comme étant actif lorsque la valeur du rapport T/C est égale ou supérieure à 125 % selon le protocole retenu. Il est toxique lorsque cette valeur est égale ou inférieure à 85 %. Le produit est également considéré comme toxique lorsque la variation pondérale, exprimée en grammes et calculée par la formule : Poids moyen du lot mesuré au jour 5 -poids moyen du même lot au jour 1 est égale ou inférieure à - 4 g.

a) Test de la leucémie P 388 (GERAN et al., Cancer Chemother, 1972, 2, 07-57).

Les essais sont effectués avec des souris hybrides CDF 1 inoculées par voie intrapéritonéale avec $10^6$ cellules leucémiques de la lignée P 388. Le composé à tester est administré pendant 5 jours (J1-J5). Le contrôle positif du test est le 5-fluorouracile qui est administré dans les mêmes conditions. Le nombre d'animaux survivants est évalué au jour J 40. Un composé est considéré comme étant actif lorsque le rapport T/C est supérieur à 127 %.

| Composé | Dose mg/kg | Variation pondérale en g | Jour médian de survie | T/C % | Nombre de survivants |
|---|---|---|---|---|---|
| Dérivé N°1 | 5 | - 0,4 | 14,7 | 142 | 0 |
| SR 25439 A | 10 | - 0,7 | 15,87 | 154 | 0 |
| | 20 | - 1,2 | 14,62 | 142 | 0 |
| Contrôle négatif | | - 0,05 | I0,3 | - | 0 |
| Contrôle positif 5 - Fluoro-uracile | 20 | - 1,3 | 20,66 | 200 | 0 |
| Dérivé N°3 | 5 | + 1,0 | 16,0 | 155 | 0 |
| SR 25440 A | 10 | - 0,6 | 15,2 | 147 | 0 |
| | 20 | - 2,2 | 15,4 | 149 | 0 |
| Contrôle négatif | - | + 1,7 | 10,3 | - | 0 |
| Contrôle positif 5 - Fluoro-uracile | 20 | - 0,1 | 19,0 | 184 | 0 |

| Composé | Dose mg/kg | Variation pondérale en g | Jour médian de survie | T/C % | Nombre de survivants |
| --- | --- | --- | --- | --- | --- |
| Dérivé N°4<br>SR 25648 A | 2,5<br>5,0<br>10,0<br>20,0<br>40,0 | + 1,4<br>+ 2,3<br>+ 1,3<br>+ 1,1<br>+ 3,8 | 14,25<br>14,7<br>15,0<br>15,1<br>7,2 | 129<br>133<br>136<br>137<br>65 | 0<br>0<br>0<br>0<br>0 |
| Contrôle négatif | - | + 1,7 | 11,05 | - | 0 |
| Contrôle positif 5 - Fluoro-uracile | 20 | + 0,5 | 19 | 186 | 0 |
| Dérivé N°5<br>SR 25525 A | 10<br>20<br>40 | - 0,6<br>+ 0,8<br>- 2,1 | 15,6<br>15<br>9,8 | 128<br>124<br>80 | 0<br>0<br>0 |
| Contrôle négatif | - | + 2,1 | 12,1 | - | 0 |
| Contrôle positif 5 - Fluoro-uracile | 20 | + 1,3 | 18 | 149 | 0 |
| Dérivé N°11<br>SR 25527 A | 1,25<br>2,5<br>5,0<br>15,0 | + 2,4<br>+ 1,6<br>+ 0,6<br>- 1,9 | 15,9<br>17,0<br>17,3<br>16,4 | 142<br>152<br>154<br>146 | 0<br>0<br>0<br>1 |
| Contrôle négatif | - | + 2,1 | 11,2 | - | 0 |
| Contrôle positif 5 - Fluoro-uracile | 20 | + 1,3 | 18,9 | 168 | 0 |

| Composé | Dose mg/kg | Variation pondérale en g | Jour médian de survie | T/C % | Nombre de survivants |
|---|---|---|---|---|---|
| Dérivé N°12 SR 25724 A | 0,625<br>1,25<br>2,5<br>5<br>10 | + 1,0<br>+ 0,1<br>- 0,3<br>- 1,2<br>- 1,7 | 13,9<br>14,1<br>15,25<br>16,7<br>15,25 | 129<br>130<br>141<br>155<br>141 | 0<br>0<br>0<br>0<br>0 |
| Contrôle négatif | | - 0,45 | 10,8 | - | 0 |
| Contrôle positif 5 - Fluoro-uracile | 20 | - 1,7 | 19 | 176 | 0 |
| Dérivé N°14 SR 25758 A | 2,5<br>5 | + 1,0<br>+ 1,7 | 14,0<br>14,0 | 127<br>127 | 0<br>0 |
| Contrôle négatif | - | + 0,9 | 11,0 | - | 0 |
| Contrôle positif 5 - Fluoro-uracile | 20 | - 1,4 | 19,3 | 175 | 0 |
| Dérivé N°15 SR 25767 A | 2,5<br>5 | - 0,9<br>- 1,1 | 13,25<br>13,9 | 124<br>130 | 0<br>0 |
| Contrôle négatif | - | + 0,9 | 11,0 | - | 0 |
| Contrôle positif 5 - Fluoro-uracile | 20 | - 1,4 | 19,3 | - | 0 |
| Dérivé N°16 SR 25768 A | 2,5<br>5<br>10<br>20 | + 0,2<br>- 1,7<br>- 2,5<br>- 4,2 | 15,0<br>14,25<br>13,9<br>8,2 | 140<br>133<br>130<br>77 | 0<br>0<br>0<br>0 |
| Contrôle négatif | - | - 0,3 | 10,7 | - | 0 |
| Contrôle positif 5 - Fluoro-uracile | 20 | - 1,6 | 19,25 | 180 | 0 |

b) Test de la leucémie L 1210 (GERAN et al., Cancer Chemother, 1972, 2, 07-57).

Les essais sont effectués avec des souris hybrides $CDF_1$ inoculées avec $10^5$ cellules leucémiques de la lignée L 1210. Le composé à tester est administré pendant 5 jours ($J_1$-$J_5$).Le contrôle positif du test est le 5-fluorouracile administré dans les mêmes conditions. Le nombre d'animaux survivants est évalué au jour J 30. Un composé est considéré comme étant actif lorsque le rapport T/C est supérieur ou égal à 125 %.

| Composé | Dose mg/kg | Variation pondérale en g | Jour médian de survie | T/C % | Nombre de survivants |
|---|---|---|---|---|---|
| Dérivé N°3 SR 25440 A | 5<br>10 | + 1,6<br>+ 1,4 | 10,6<br>11,3 | 129<br>138 | 0<br>0 |
| Contrôle négatif | | + 1,7 | 8,2 | - | 0 |
| Contrôle positif 5 - Fluoro - uracile | 20 | + 0,8 | 15,7 | 191 | 0 |
| Dérivé N°4 SR 25648 A | 2,5<br>5<br>10<br>20 | + 0,5<br>+ 1,1<br>- 3,1<br>- 1,4 | 10,3<br>13,0<br>11,25<br>10,1 | 126<br>158<br>137<br>123 | 0<br>0<br>0<br>0 |
| Contrôle négatif | - | + 1,7 | 8,2 | - | 0 |
| Contrôle positif 5 - Fluoro-uracile | 20 | + 0,8 | 15,7 | 191 | 0 |
| Dérivé N°11 SR 25527 A | 1,25<br>2,5<br>5<br>7,5<br>10<br>20<br>30 | + 0,3<br>- 0,6<br>+ 0,3<br>- 0,3<br>- 0,4<br>- 1,7<br>- 4,8 | 10,9<br>12,75<br>14,25<br>12,3<br>12,25<br>11,7<br>8,3 | 122<br>143<br>160<br>138<br>138<br>131<br>93 | 0<br>0<br>0<br>0<br>0<br>0<br>0 |
| Contrôle négatif | | + 0,6 | 8,6 | - | 0 |
| Contrôle positif - 5 Fluoro-uracile | 20 | + 1,1 | 15,5 | 174 | 0 |

| Composé | Dose mg/kg | Variation pondérale en g | Jour médian de survie | T/C % | Nombre de survivants |
|---|---|---|---|---|---|
| Dérivé N°15 SR 25767 A | 10 | + 0,2 | 10,2 | 129 | 0 |
| Contrôle négatif | | + 0,25 | 7,92 | - | 0 |
| Contrôle positif 5 - Fluoro-uracile | 20 | - 0,2 | 14,5 | 183 | 0 |
| Dérivé N°16 SR 25768 A | 5<br>10<br>20 | - 0,7<br>- 1,5<br>- 3,2 | 10<br>10,4<br>10,2 | 126<br>131<br>129 | 0<br>0<br>0 |
| Contrôle négatif | | + 0,25 | 7,92 | - | 0 |
| Contrôle positif 5 - Fluoro-uracile | 20 | - 0,02 | 14,5 | 183 | 0 |

c) Test du mélanome B 16 (GERAN et al., Cancer Chemother, 1972, 2,07-57).

Les essais sont effectués avec des souris hybrides $BDF_1$ inoculées avec 0,5 ml d'un homogénat de mélanome B 16 réalisé à partir de 1 g de tumeur dans 10 ml de sérum physiologique. Le composé à tester est administré pendant 9 jours ($J_1$-$J_9$).Le contrôle positif du test est le Cis-Platine administré dans les mêmes conditions. Le nombre d'animaux survivants est évalué au jour J 60. Un composé est considéré comme étant actif lorsque le rapport T/C est supérieur ou égal à 125 %.

| Composé | Dose mg/kg | Variation pondérale en g | Jour médian de survie | T/C % | Nombre de survivants |
|---|---|---|---|---|---|
| Dérivé N°3<br>SR 25440 A | 2,5 | - 0,1 | 36 | 138 | 0 |
| Contrôle négatif | - | - 0,15 | 26 | - | 0 |
| Contrôle positif Cis-Platine | 1 | - 0,8 | 37 | 142 | 0 |
| Dérivé N°11<br>SR 25527 A | 0,625<br>1,25<br>2,5<br>5,0 | + 0,2<br>+ 0,2<br>+ 1,0<br>- 1,0 | 29<br>40<br>38<br>31 | 123<br>170<br>162<br>132 | 0<br>1<br>2<br>0 |
| Contrôle négatif | - | + 1,9 | 23,5 | - | 0 |
| Contrôle positif Cis-Platine | 1 | + 0,8 | 37,0 | 157 | 0 |
| Dérivé N°14<br>SR 25758 A | 5<br>20 | - 0,2<br>- 1,3 | 34,25<br>38,75 | 123<br>139 | <br>0 |
| Contrôle négatif | | - 0,7 | 27,93 | - | 0 |
| Contrôle positif Cis-Platine | 1 | - 0,7 | 39 | 140 | 0 |

Les études toxicologique et pharmacologique qui viennent d'être rapportées ont mis en évidence les intéressantes propriétés antitumorales des composés de l'invention qui les rendent très utiles en

thérapeutique.

Le médicament de l'invention peut être présenté pour l'administration oral sous forme de comprimés, comprimés dragéifiés, capsules, gouttes ou sirop. Il peut aussi être présenté pour l'administration rectale sous forme de suppositoires et pour l'administration parentérale sous forme de soluté injectable par voie intramusculaire ou intraveineuse.

Chaque dose unitaire contient avantageusement de 0,010 g à 0,500 g associés à des excipients et véhicules convenables, les doses administrables journellement peuvent varier de 0,010 g à 3,00 g de principe actif, en fonction du poids du malade, de son âge et de la sévérité de son état.

On donnera ci-après à titre d'exemple non limitatif, quelques formulations pharmaceutiques du médicament de l'invention.

1) Comprimés dragéifiés
Dérivé No 11 0,250 g
excipient : lévilite, talc, stéarate de magnésium, gomme laque, gomme arabique, saccharose, oxyde de titane.

2) Comprimés
Dérivé No 3 0,300 g
excipient : gomme arabique, lactose, stéarate de magnésium, talc.

3) Capsules
Dérivé No 12 0,500 g
excipient : stéarate de magnésium, amidon de maïs, talc.

4) Suppositoires
Dérivé No 1 0,250 g
excipient : triglycérides semi-synthétiques qsp 1 suppositoire.

5) Soluté injectable
Dérivé No 11 0,500 g
excipient : solvant isotonique qsp 5 ml.

Pour ses propriétés antitumorales, le médicament de l'invention est indiqué dans le traitement des tumeurs solides et de leurs métastases ainsi que dans le traitement des tumeurs ascitiques.

L'invention comprend également les nouveaux dérivés de la chloro-6 11-H indolo[3,2-c]quinoléine de formule III, utiles en tant qu'intermédiaires de synthèse dans la préparation de composés (I), et à un procédé pour les préparer.

Ce procédé de préparation des composés de formule III ci-dessus est caractérisé en ce que :

a) par action d'une alcoyl($C_1$-$C_4$)oxy-4 phénylhydrazine sur la nitro-2 acétophénone selon la méthode de cyclisation dite de Fisher, on obtient un alcoyloxy-5 (nitro-2 phényl)-2 indole de formule IVa :

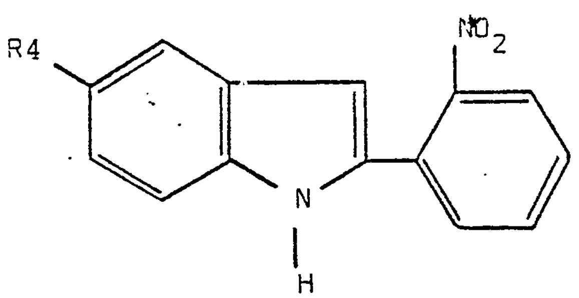

IVa

dans laquelle $R_4$ est un groupe alcoyloxy en $C_1$-$C_4$,

b) par action d'un halogénure de formule $R'_1$-hal ($R'_1$ représentant un groupe alcoyle en $C_1$-$C_4$ ou le groupe tétrahydropyran-2-yle, et hal représentant le chlore, le brome ou l'iode) on prépare les composés de formule IVb:

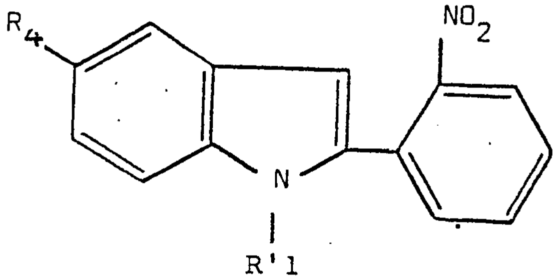

IVb

dans laquelle R'1 représente un groupe alcoyle en $C_1$-$C_4$ ou le groupe tétrahydropyranne-2-yle et $R_4$ un groupe alcoxy en $C_1$-$C_4$,

c) par réduction catalytique du dérivé de formule IVb on obtient un dérivé aminé de formule V :

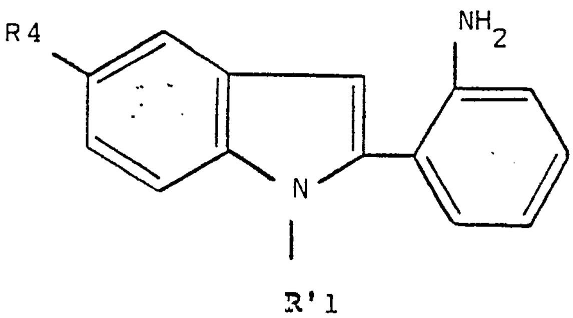

V

dans laquelle R'1 et $R_4$ ont la même signification que dans la formule IVb,

d) par réaction de chloroformiate d'alcoyle en $C_1$-$C_4$ sur un composé de formule V on obtient un carbamate de formule :

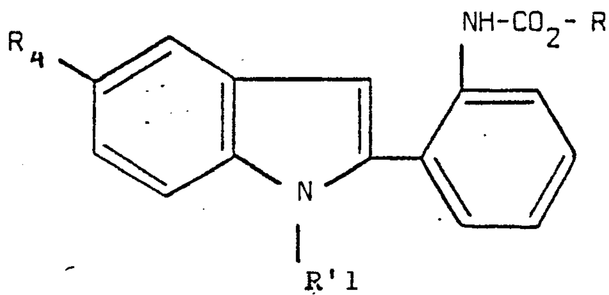

VI

dans laquelle R'1 et $R_4$ ont les mêmes significations que dans la formule IVb, et R représente un radical alcoyle en $C_1$-$C_4$,

e) par cyclisation thermique du carbamate de formule VI, on obtient une 5-H, 11-H, indolo[3,2-c]quinolone-6 de formule VII :

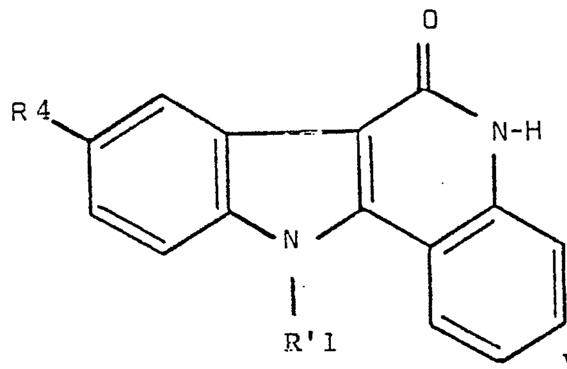

VII

dans laquelle $R'_1$ et $R_4$ ont les mêmes significations que dans la formule IVb,

f) par action d'un agent chlorurant on effectue la chloration d'un composé de formule VII pour obtenir une alcoyloxy-8 chloro-6 11-H indolo[3,2-c]quinoléine de formule III :

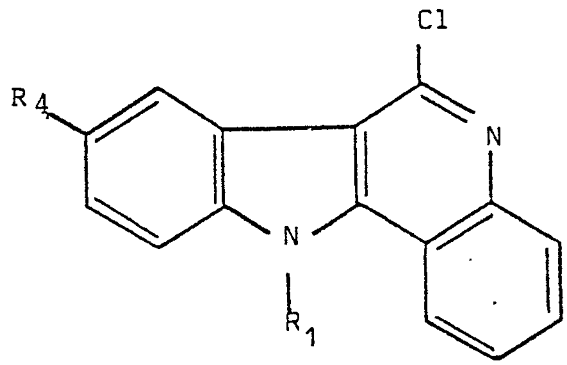

III

dans laquelle $R_1$ représente soit un groupe alcoyle en $C_1$-$C_4$, soit, lorsque dans le composé de formule VII mis en oeuvre dans la réaction de chloration $R'_1$ représente le groupe tétrahydropyranne-2-yle, un hydrogène.

La formation des composés de formule IVb s'effectue avantageusement par action de l'halogénure approprié sur le dérivé sodé d'un (nitro-2 phényl)-2 alcoyloxy-5 indole, obtenu, par exemple, par action de l'hydrure de sodium, à une température comprise entre 0° C et 80° C dans un solvant inerte aprotique polaire tel que le N,N-diméthylformamide.

La réduction d'un composé de formule IVb est effectuée par action de $H_2$ à pression ordinaire à une température comprise entre 20° C et 60° C en présence d'un catalyseur tel que la charbon palladié.

L'obtention d'un composé de formule VI s'effectue à une température comprise entre 40° C et 80° C en présence d'une base telle que la pyridine. La cyclisation d'un composé de formule VI est réalisée à une température comprise entre 150° C et 210° C dans un solvant inerte tel que l'éther diphénylique.

La réaction de chloration qui conduit au composé de formule III s'effectue avantageusement par action de l'oxychlorure de phosphore porté au reflux. Les exemples non limitatifs suivants sont donnés à titre d'illustration de l'invention.

EXEMPLE 19.

Préparation de la chloro-6 méthoxy-8 méthyl-11 11-H indolo [3,2-c]quinoléine (composé de formule III dans laquelle $R_1$ = $-CH_3$, $R_4$ = $-O-CH_3$).

a) Préparation du méthoxy-5 (nitro-2 phényl)-2 indole (composé de formule IVa)

On introduit dans un réacteur de 250 ml, 18 g (0,13 mole) de paraméthoxyphénylhydrazine, 21,5 g (0,12 mole) de nitro-2 acétophénone, 160 ml d'éthanol absolu et 2,2 ml d'acide acétique. Le milieu réactionnel est agité sous atmosphère d'azote, à l'abri de la lumière et à température ambiante pendant 20 heures. L'hydrazone obtenue précipite, elle est filtrée, lavée à l'éther isopropylique glacé et séchée. On isole ainsi 27,3 g de cristaux orange.

Rendement : 80 % ; F : 133° C.

Puis dans un réacteur d'un litre, on chauffe sous azote 400 g d'ester éthylique de l'acide polyphosphorique (P.P.E.) à 60° C. On additionne ensuite par frac tions 41 g d'hydrazone préparée ci-dessus et le milieu réactionnel est porté à 60-65° C durant 5 heures sous agitation et à l'abri de la lumière. Après retour à la

température ordinaire, on verse le milieu réactionnel dans 2 litres du mélange eau-glace. Après 15 minutes d'agitation, on extrait au dichlorométhane. La phase organique est lavée à l'eau, séchée et concentrée sous pression réduite.

L'huile brune obtenue est purifiée sur colonne de silice (éluant : dichlorométhane). On obtient ainsi 25 g de cristaux jaunes de méthoxy-5 (nitro-2 phényl)-2 indole.

Rendement : 65 % ; F : 110°C.

Calc. pour : $C_{15}H_{12}N_2O_3$ : C 67,15 ; H 4,51 ; N 10,44 %

Trouvé : C 67,28 ; H 4,53 ; N 10,37 %

b) Préparation du méthoxy-5 méthyl-1 (nitro-2 phényl)-2 indole, (composé de formule IVb).

A une suspension de 9,84 g d'hydrure de sodium à 50 % dans l'huile (0,205 mole) dispersée dans 100 ml de N,N-diméthylformamide, on ajoute goutte à goutte et sous courant d'azote une solution de 50 g (0,186 mole) de méthoxy-5 (nitro-2 phényl)-2 indole dans 300 ml de N,N-diméthylformamide. On chauffe le milieu à 35°C jusqu'à cessation du dégagement d'hydrogène. Puis le courant d'azote est coupé et 50 g (0,352 mole) d'iodure de méthyle sont ajoutés lentement. Après cette addition, le milieu est chauffé une heure à 50°C. Le N,N-diméthylformamide est alors évaporé sous pression réduite. Le produit brut obtenu est repris par 100 ml d'eau, filtré et recristallisé dans le méthanol. On isole ainsi 49 g de méthoxy-5 méthyl-1 (nitro-2 phényl)-2 indole sous forme de cristaux orange.

Rendement : 93 % ; F : 144°C.

Calc. pour : $C_{16}H_{14}N_2O_3$ : C 68,07 ; H 5,00 ; N 9,92 %

Trouvé : C 68,16 ; H 4,86 ; N 9,99 %

c) Préparation de l'(amino-2 phényl)-2 méthoxy-5 méthyl-1 indole (composé de formule V).

On solubilise à chaud 30 g (0,106 mole) de méthoxy-5 méthyl-1 (nitro-2 phényl)-2 indole dans 1400 ml d'un mélange d'acide acétique et de méthanol (environ 40/60). Après retour à température ordinaire et sous atmosphère d'azote, on ajoute 3 g de charbon palladié à 5 %. Puis on réalise l'hydrogénation sous pression normale. Après absorption du volume théorique d'hydrogène (environ 6700 ml), on filtre le catalyseur qui est lavé au méthanol. Les phases organiques réunies sont concentrées sous pression réduite. L'(amino-2 phényl)-2 méthoxy-5 méthyl-1 indole est recristallisé dans le méthanol et on isole ainsi 15,7 g de produit pur.

Rendement : 58,5 % ; F : 138°C.

Calc. pour : $C_{16}H_{16}N_2O$ ; 0,25 $H_2O$ : C 74,82 ; H 6,34 ; N 10,91 %

Trouvé : C 74,65 ; H 6,22 ; N 11,10 %

d) Préparation de l'(éthoxycarbonylamino-2 phényl)-2 méthoxy-5 méthyl-1 indole (composé de formule VI).

Dans 200 ml de tétrahydrofuranne anhydre, on introduit 5,3 g de pyridine (0,72 mole) et 15 g de l'(amino-2 phényl)-2 méthoxy-5 méthyl-1 indole (0,06 mole). Le milieu est porté au reflux puis on ajoute goutte à goutte 9,2 g de chloroformiate d'éthyle (0,72 mole) dilués dans 50 ml de tétrahydrofuranne. Le milieu réactionnel est ensuite chauffé à 60°C-70°C pendant 2 heures 30. Après refroidissement et filtration du chlorhydrate de pyridine, la solution organique est concentrée sous pression réduite. L'huile obtenue est reprise par du dichlorométhane. La phase organique est lavée à l'eau, séchée et concentrée sous pression réduite. Le précipité obtenu est lavé par un mélange d'éthanol et d'eau. On isole ainsi 16,6 g d'(éthoxycarbonylamino-2 phényl)-2 méthoxy-5 méthyl-1 indole.

Rendement : 86 % ; F : 98°C.

Calc. pour : $C_{19}H_{20}N_2O_3$ : C 70,35 ; H 6,22 ; N 8,64 %

Trouvé : C 70,33 ; H 6,33 ; N 8,63 %

IR (KBr) : νc = o : 1730 $cm^{-1}$

e) Préparation de la méthoxy-8 méthyl-11 5-H, 11-H indolo [3,2-c]quinolone-6 (composé de formule VII).

On solubilise 15 g d'(éthoxycarbonylamino-2 phényl)-2 méthoxy-5 méthyl-1 indole (0,046 mole) dans 150 ml d'éther diphénylique. Le milieu réactionnel est ensuite chauffé pendant 2 heures à 200°C. Après refroidissement, la méthoxy-8 méthyl-11 5-H 11-H indolo[3,2-c] quinolone-6 précipite. Elle est filtrée et lavée à l'éther éthylique. On isole ainsi 12,5 g de produit pur.

Rendement : 97 % ; F : > 260°C.

Calc. pour : $C_{17}H_{14}N_2O_2$ : C 73,39 ; H 5,01 ; N 10,07

Trouvé : C 73,18 ; H 5,07 ; N 10,16

IR (KBr) : νc = o : 1650 $cm^{-1}$

f) Préparation de la chloro-6 méthoxy-8 méthyl-11 11-H indolo[3,2-c]quinoléine.

On dissout 20 g de méthoxy-8 méthyl-11 5-H 11-H indolo[3,2-c]quinolone-6 dans 750 ml d'oxychlorure de phosphore. Le milieu réactionnel est porté au reflux pendant 2 heures. Après refroidissement le chlorhydrate de la chloro-6 méthoxy-8 méthyl-11 11-H indolo[3,2-c]quinoléine précipite partiellement. L'excès d'oxychlorure de phosphore est distillé sous pression réduite. Le précipité est alors dispersé dans l'eau, le chlorhydrate est filtré, lavé à l'acétone puis à l'éther éthylique, puis il est neutralisé par une solution aqueuse de soude N.

La base est extraite par le dichlorométhane. La phase organique est séchée puis concentrée sous pression réduite. On obtient ainsi 18,7 g de chloro-6 méthoxy-8 méthyl-11 11-H indolo[3,2-c]quinoléine.

Rendement : 87,6 % ; F : 200° C.
Calc. pour : $C_{17}H_{13}N_2O$ Cl : C 68,80 ; H 4,42 ; N 9,44
Trouvé : C 68,55 ; H 4,22 ; N 9,41 %
IR (KBr) : absence de νc = O.

EXEMPLE 20

Préparation de la chloro-6 méthoxy-8 11-H indolo[3,2-c]quinoléine (composé de formule III dans laquelle $R_1$ = H, $R_4$ = -$OCH_3$).

a) Préparation du méthoxy-5 (nitro-2 phényl)-2 (tétrahydropyran-2 yl)-1 indole, (composé de formule IVb).

Dans 300 ml de N,N-diméthylformamide anhydre, on ajoute 100 g (0,37 mole) de méthoxy-5 (nitro-2 phényl)-2 indole. Puis, sous courant d'azote et à température ordinaire, on introduit progressivement 18 g (0,37 mole) d'hydrure de sodium à 50 % en suspension dans l'huile. Le milieu réactionnel est ensuite chauffé à 50° C pendant 15 minutes environ jusqu'à arrêt du dégagement d'hydrogène. Après refroidissement à 10° C, on ajoute goutte à goutte 68 g (0,5 mole) de chloro-2 tétrahydropyranne fraîchement distillé. On maintient sous agitation le milieu réactionnel à 20° C pendant 12 heures. Le milieu réactionnel est alors versé dans 500 ml d'eau, puis extrait au dichlorométhane. La phase organique est lavée à l'eau, séchée, concentrée sous pression réduite. Le précipité obtenu est lavé à l'hexane puis à l'éther isopropylique. On isole ainsi 77 g de méthoxy-5 (nitro-2 phényl)-2 (tétrahydropyran-2 yl)-1 indole sous forme de poudre jaune qui est engagée dans l'étape suivante sans autre purification.
Rendement : 59 % ; F : 136° C.
Calc. pour $C_{20}H_{20}N_2O_4$ ; 0,2 $H_2O$ : C 67,48 ; H 5,78 ; N 7,88%
Trouvé : C 67,46 ; H 5,79 ; N 7,63%

b) Préparation de l'(amino-2 phényl)-2 méthoxy-5 (tétrahydropyran-2 yl)-1 indole (composé de formule V).

En partant de méthoxy-5 (nitro-2 phényl)-2 (tétra hydropyran-2 yl)-1 indole en solution méthanolique et selon le mode opératoire décrit pour la réduction du méthoxy-5 méthyl-1 (nitro-2 phényl)-2 indole, (exemple 19 - c) on obtient l'(amino-2 phényl)-2 méthoxy-5 (tétrahydropyran-2 yl)-1 indole.
Rendement : 34 % ; F : 150° C.
Calc. pour $C_{20}H_{22}N_2O_2$ ; $O^2$ $H_2O$ : C 73,70 ; H 6,93 ; N 8,60 %
Trouvé : C 73,70 ; H 6,97 ; N 8,64 %

c) Préparation de l'(éthoxycarbonylamino-2 phényl)-2 méthoxy-5 (tétrahydropyran-2 yl)-1 indole (composé de formule VI).

En partant d'(amino-2 phényl)-2 méthoxy-5 (tétrahydropyran-2 yl)-1 indole et selon le mode opératoire décrit pour la préparation d'(éthoxycarbonylamino-2 phényl)-2 méthoxy-5 méthyl-1 indole, (exemple 19d), on obtient l'(éthoxycarbonylamino-2 phényl)-2 méthoxy-5 (tétrahydropyran-2 yl)-1 indole.
Rendement : 76 % après recristallisation dans l'éthanol à 95 % ; F : 114° C.
Calc. pour $C_{23}H_{26}N_2O_4$ ; 0,2 $H_2O$ : C 69,39 ; H 6,69 ; N 7,04 %
Trouvé : C 69,34 ; H 6,84 ; N 6,89 %
IR (KRr) : νc = o 1730 $cm^{-1}$

d) Préparation de la méthoxy-8 (tétrahydropyran-2 yl)-11 5-H, 11-H indolo[3,2-c]quinolone-6 (composé de formule VII).

On solubilise 53 g d'(éthoxycarbonylamino-2 phényl)-2 méthoxy-5 (tétrahydropyran-2 yl)-1 indole (0,13 mole) dans 700 ml d'éther diphénylique. Puis le milieu réactionnel est chauffé à 175° C pendant 6 heures. Après concentration sous pression réduite de l'éther diphénylique jusqu'à un volume d'environ 200 ml, le produit attendu précipite. Il est filtré et lavé à l'éther éthy lique. On obtient ainsi 23,5 g de méthoxy-8 (tétrahydropyran-2 yl)-11 5-H, 11-H indolo[3,2-c]quinolone-6.
Rendement : 50 % ; F > 260° C.
Calc. pour $C_{21}H_{20}N_2O_3$ : C 72,39 ; H 5,79 ; N 8,04 %
Trouvé : C 72,20 ; H 5,81 ; N 7,91 %
IR (KBr) : νc = o : 1645 $cm^{-1}$
RMN $H_1$ [$(CD_3)_2$ SO] ; δ 1,2-2,5 (m, 3 x 2H, $CH_2$-cyclique) 3,4-3,6 (m, 2H, $CH_2$-O) ; 3,8 (s, 3H, $OCH_3$) ; 4-4,3 (m, 1H, O-CH-N) ; 6,8-8 (m, 7H, protons aromatiques).

e) Préparation de la chloro-6 méthoxy-8 11-H indolo [3,2-c]quinoléine.

On dissout 2 g de méthoxy-8 (tétrahydropyran-2 yl) 5H 11-H indolo(3,2-c]quinolone-6 dans 30 ml d'oxychlorure de phosphore fraîchement distillé. Puis le milieu réactionnel est porté au reflux, sous atmosphère anhydre, pendant 1 H 15 mn. L'excès d'oxychlorure de phosphore est distillé sous pression réduite puis le chlorhydrate de chloro-6 méthoxy-8 11-H indolo[3,2-c]quinoléine est neutralisé par une solution aqueuse de soude N. La base est extraite au dichlorométhane. La phase organique est lavée à l'eau, séchée et concentrée sous pression réduite. Le produit brut ainsi obtenu est purifié sur colonne de silice (éluant : dichlorométhane-méthanol, 95/5).

On obtient ainsi 0,87 g de chloro-6 de méthoxy-8 11-H indolo[3,2-c]quinoléine.
Rendement : 53 % ; F : 248°C.
Calc. pour $C_{16}H_{11}N_2O$ Cl ; 0,3 $H_2O$ : C 66,69 ; H 4,06 ; N 9,72 %
Trouvé : C 66,82 ; H 3,73 ; N 9,39 %
IR (KBr) : absence de νc = O.
RMN $H_1$ [$(CD_3)_2$ SO] ; δ 3,95 (S, 3H, $OCH_3$) ; 7-8,2 (m, 7H, protons aromatiques).

## Revendications

1. Composés de formule générale :

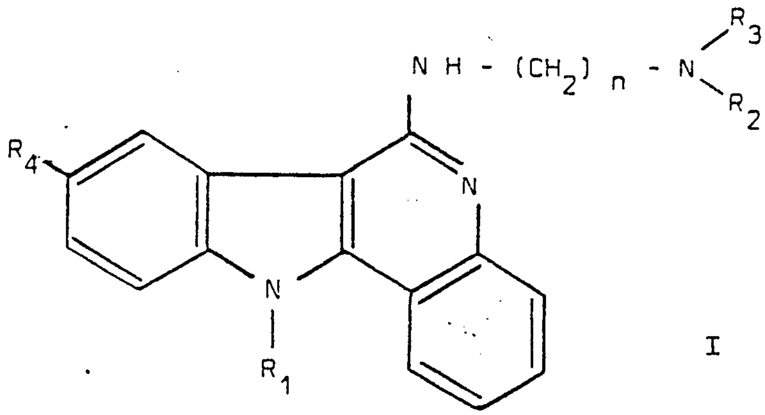

I

dans laquelle n représente un nombre entier de 2 à 4, $R_1$ représente l'hydrogène ou un groupe alcoyle en $C_1$-$C_4$, $R_2$ et $R_3$ identiques ou différents représentent l'hydrogène ou un groupe alcoyle en $C_1$-$C_4$ ou bien $R_2$ et $R_3$ forment ensemble et avec l'atome d'azote auquel ils sont rattachés un hétérocycle saturé pouvant comporter un second hétéroatome tel que l'oxygène, le soufre ou l'azote, $R_4$ représente un groupe hydroxyle ou alcoyloxy en $C_1$-$C_4$, et leurs sels d'addition avec les acides minéraux ou organiques pharmaceutiquement acceptables ainsi que les formes tautomères lorsqu'elles existent.

2. La (diéthylamino-3 propylamino)-6 méthoxy-8 méthyl-11 11-H indolo [3,2-c]quinoléine, et ses sels pharmaceutiquement acceptables.

3. La (diéthylamino-3 propylamino)-6 hydroxy-8 méthyl-11 11-H indolo[3,2-c]quinoléine, et ses sels pharmaceutiquement acceptables.

4. La (diéthylamino-3 propylamino)-6 hydroxy-8 11-H indolo[3,2-c]quinoléine et ses sels pharmaceutiquement acceptables?

5. La (diméthylamino-3 propylamino)-6 hydroxy-8 11-H indolo[3,2-c]quinoléine, et ses sels pharmaceutiquement acceptables.

6. Procédé de préparation des composés selon la revendication 1, caractérisé en ce que :

a) par réaction de l'oxychlorure de phosphore sur une 11-H N-oxyde indolo[3,2-c]quinoléine de formule II :

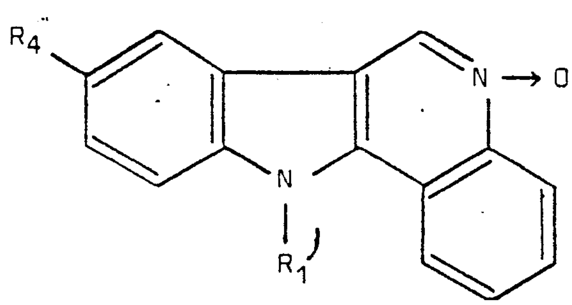

II

dans laquelle R'$_1$ représente un groupe alcoyle en $C_1$-$C_4$ ou le groupe tétrahydropyrannyle-2 et $R_4$ représente un groupe alcoyloxy en $C_1$-$C_4$, puis le cas échéant par action d'une solution aqueuse d'acide chlorhydrique sur les composés tétrahydropyrannylés en position 11 ainsi obtenus pour préparer les dérivés non substitués en position 11 ($R_1$ = H), on obtient les chloro-6 11-H indolo[3,2-c]quinoléines correspondantes de formule III, isolées sous forme de base ou de chlorhydrate,

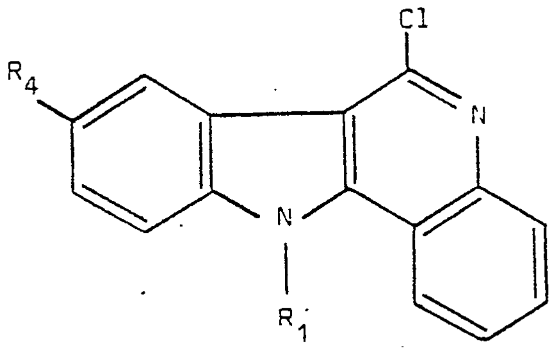

III

dans laquelle $R_1$ a la même signification que dans la formule I et $R_4$ représente un groupe alcoyloxy en $C_1$-$C_4$,

b) on condense le composé de formule III avec une amine de formule IV :

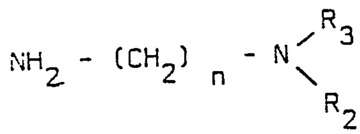

IV

dans laquelle n, $R_2$ et $R_3$ ont les significations précitées pour la formule I, pour obtenir le composé de formule I recherché dans laquelle $R_4$ représente un groupe alcoyloxy en $C_1$-$C_4$, et le cas échéant

c) on prépare les composés de formule I dans lesquels $R_4$ représente un hydroxyle par désalcoylation des composés de formule I dans lesquels $R_4$ représente un radical alcoyloxy en $C_1$-$C_4$.

7. Procédé selon la revendication 2, caractérisé en ce que la formation des composés chlorés de formule III s'effectue à une température comprise entre 10° C et la température de reflux de l'oxychlorure de phosphore.

8. Procédé selon la revendication 2 ou 3, caractérisé en ce que la réaction d'amination des composés de formule III s'effectue, éventuellement dans un solvant inerte tel que l'éther diphénylique, à une température comprise entre 120° C et 200° C.

9. Médicament caractérisé en ce qu'il contient, à titre de principe actif, un dérivé selon l'une des revendications 1 à 5 ou l'un de ses sels pharmaceutiquement acceptables.

10. Médicament selon la revendication 9, caractérisé en ce que chaque dose unitaire contient de 0,010 à 0,500 g de principe actif.

11. Composés de formule générale :

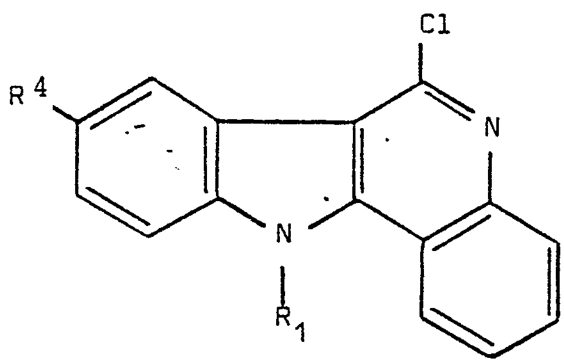

III

dans laquelle $R_1$ représente l'hydrogène ou un radical alcoyle en $C_1$-$C_4$ et $R_4$ représente un radical alcoyloxy en $C_1$-$C_4$, ainsi que ses formes tautomères lorsqu'elles existent.

12. La chloro-6 méthoxy-8 méthyl-11 11-H indolo [3,2-c]quinoléine.

13. La chloro-6 méthoxy-8 11-H indolo[3,2-c]quinoléine.

14. Procédé de préparation des composés de formule III, caractérisé en ce que :

a) on fait réagir une alcoyl($C_1$-$C_4$)oxy-4 phénylhydrazine sur la nitro-2 acétophénone, pour obtenir un alcoyloxy-5 (nitro-2 phényl)-2 indole de formule :

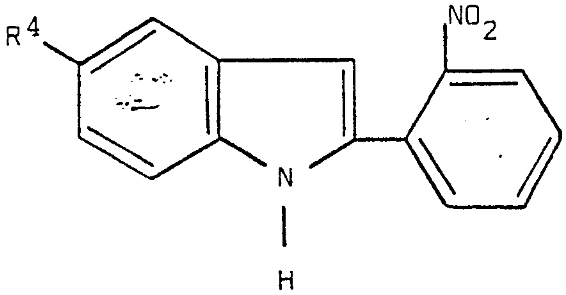

IVa

dans laquelle $R_4$ est un groupe alcoyloxy en $C_1$-$C_4$,

b) on fait réagir sur le composé IVa un halogénure $R'_1$-hal, $R'_1$ étant un groupe alcoyle en $C_1$-$C_4$ ou le groupe tétrahydropyranne-2-yle et hal étant le chlore, le brome ou l'iode, pour préparer un composé de formule IVb :

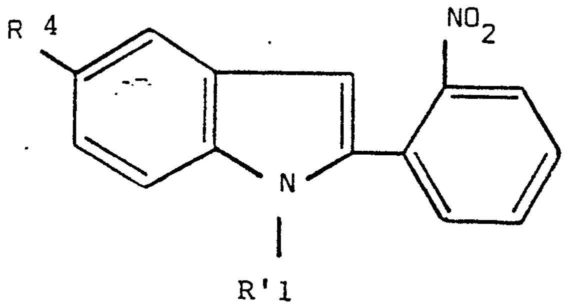

IVb

dans laquelle $R'_1$ représente un groupe alcoyle en $C_1$-$C_4$ ou le groupe tétrahydropyranne-2-yle et $R_4$ un groupe alcoxy en $C_1$-$C_4$,

c) on effectue la réduction catalytique du composé de formule IVb pour obtenir un dérivé aminé de formule V :

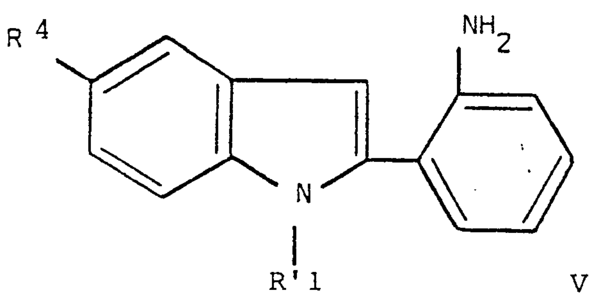

V

dans laquelle les radicaux $R'_1$ et $R_4$ ont les mêmes significations que dans la formule IVb,

d) on fait réagir un chloroformiate d'alcoyle en $C_1$-$C_4$ sur le composé de formule V pour obtenir le carbamate de formule :

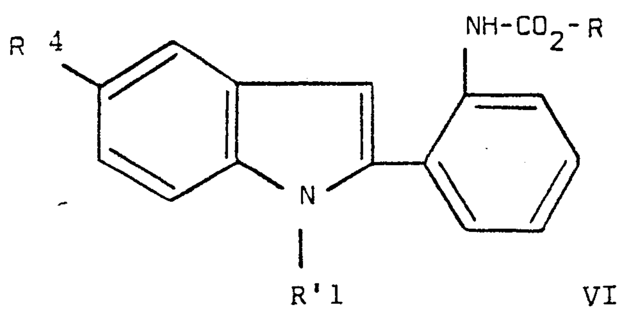

VI

dans laquelle R représente un radical alcoyle en $C_1$-$C_4$ et $R'_1$ et $R_4$ ont les mêmes significations que dans la formule IVb,

e) on soumet à la chaleur le carbamate de formule VI, pour obtenir par cyclisation une 5-H, 11-H, indolo[3,2-c]quinolone-6 de formule :

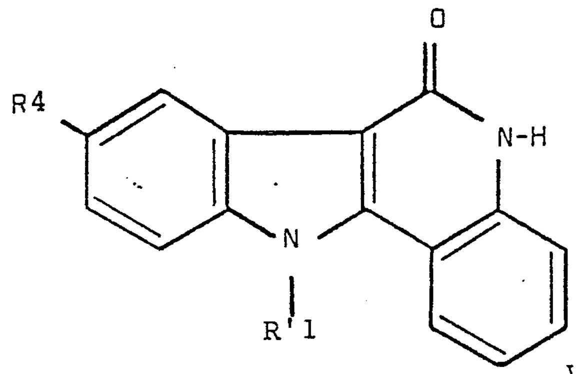

VII

dans laquelle $R'_1$ et $R_4$ ont les mêmes significations que dans la formule IVb,

f) on fait réagir un agent chlorurant sur le composé de formule VII pour obtenir le composé de formule III dans laquelle $R_1$ représente soit l'hydrogène lorsque dans le composé de formule VII mis en oeuvre le radical $R'_1$ était le groupe tétrahydropyranne-2-yle, soit un radical alcoyle en $C_1$-$C_4$.

15. Procédé selon la revendication 14, caractérisé en ce que la formation du composé de formule IVb s'effectue à une température comprise entre 0° C et 80° C dans un solvant inerte aprotique tel que le N,N-diméthylformamide.

16. Procédé selon la revendication 14 ou 15, caractérisé en ce que la réduction du composé de formule IVb est effectuée par action du $H_2$ sous pression ordinaire à une température comprise entre 20° C et 60° C en présence d'un catalyseur tel que le charbon palladié.

17. Procédé selon l'une quelconque des revendications 14 à 16, caractérisé en ce que le carbamate de formule VI est obtenu par action d'un chloroformiate d'alcoyle à une température comprise entre 40° C et 80° C en présence d'une base telle que la pyridine.

18. Procédé selon l'une quelconque des revendications 14 à 17, caractérisé en ce que la cyclisation du composé de formule VI est réalisée à une température comprise entre 150° C et 210° C dans un solvant inerte tel que l'éther diphénylique.

19. Procédé selon l'une quelconque des revendications 14 à 18, caractérisé en ce que la chloration du composé de formule VII s'effectue par action de l'oxychlorure de phosphore porté au reflux.

Revendications pour les Etats contractants suivants : AT, GR, ES

1. Procédé de préparation de composés de formule générale :

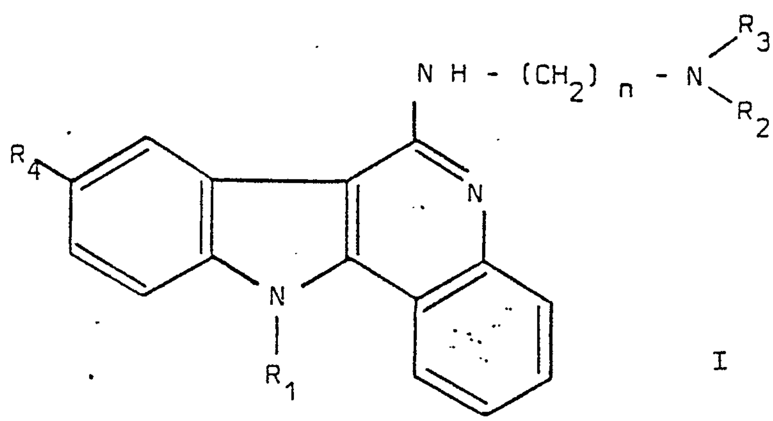

I

dans laquelle n représente un nombre entier de 2 à 4, $R_1$ représente l'hydrogène ou un groupe alcoyle en $C_1$-$C_4$, $R_2$ et $R_3$ identiques ou différents représentent l'hydrogène ou un groupe alcoyle en $C_1$-$C_4$ ou bien $R_2$ et $R_3$ forment ensemble et avec l'atome d'azote auquel ils sont rattachés un hétérocycle saturé pouvant comporter un second hétéroatome tel que l'oxygène, le soufre ou l'azote, $R_4$ représente un groupe hydroxyle ou alcoyloxy en $C_1$-$C_4$, et leurs sels d'addition avec les acides minéraux ou organiques pharmaceutiquement acceptables ainsi que les formes tautomères lorsqu'elles existent, caractérisé en ce que :

a) par réaction de l'oxychlorure de phosphore sur une 11-H N-oxyde indolo[3,2-c]quinoléine de formule II :

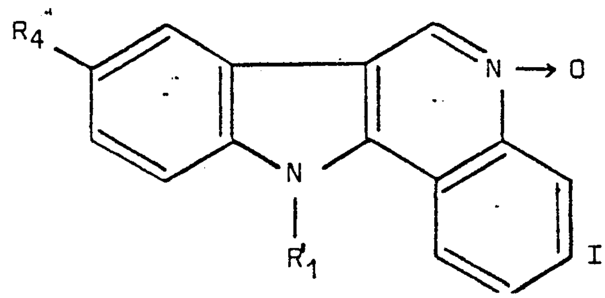

II

dans laquelle R'₁ représente un groupe alcoyle en $C_1$-$C_4$ ou le groupe tétrahydropyrannyle-2 et $R_4$ représente un groupe alcoyloxy en $C_1$-$C_4$, puis le cas échéant par action d'une solution aqueuse d'acide chlorhydrique sur les composés tétrahydropyrannylés en position 11 ainsi obtenus, pour préparer les dérivés non substitués en position 11 ($R_1$ = H), on obtient les chloro-6 11-H indolo[3,2-c]quinoléines correspondantes de formule III, isolées sous forme de base ou de chlorhydrate,

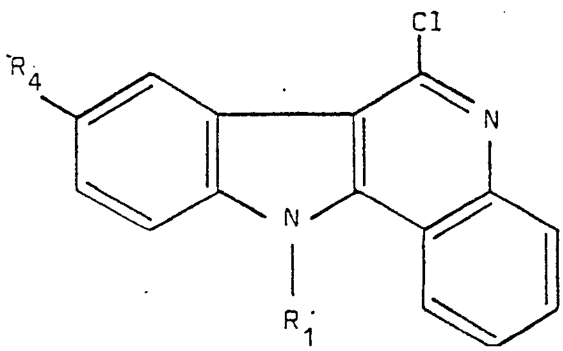

III

dans laquelle $R_1$ a la même signification que dans la formule I et $R_4$ est un groupe alcoyloxy en $C_1$-$C_4$,

b) on condense le composé de formule III avec une amine de formule IV :

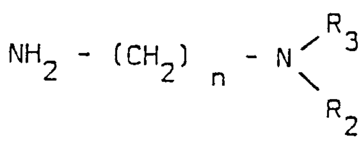

IV

dans laquelle n, $R_2$ et $R_3$ ont les significations précitées pour la formule I, pour obtenir le composé de formule I recherché dans laquelle $R_4$ représente un groupe alcoyloxy en $C_1$-$C_4$, et le cas échéant

c) on prépare les composés de formule I dans lesquels $R_4$ représente un hydroxyle par désalcoylation des composés de formule I dans lesquels $R_4$ représente un radical alcoyloxy en $C_1$-$C_4$.

2. Procédé selon la revendication 1, caractérisé en ce que la formation des composés chlorés de formule III s'effectue à une température comprise entre 10° C et la température de reflux de l'oxychlorure de phosphore.

3. Procédé selon la revendication 1 ou 2, caractérisé en ce que la réaction d'amination des composés de formule III s'effectue, éventuellement dans un solvant inerte tel que l'éther diphénylique, à une température comprise entre 120° C et 200° C.

4. Procédé de préparation d'un médicament caractérisé en ce que l'on met sous forme administrable un dérivé obtenu selon l'une des revendications 1 à 3.

5. Procédé de préparation composés de formule générale :

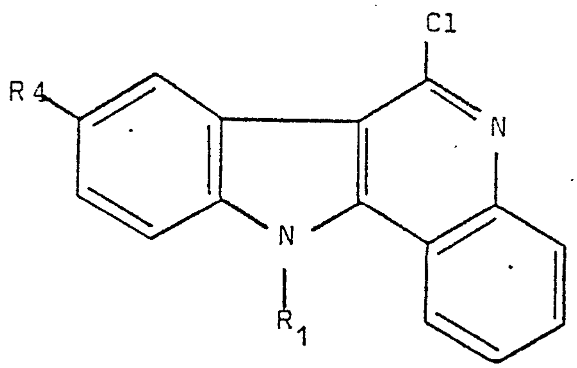

III

dans laquelle $R_1$ représente l'hydrogène ou un radical alcoyle en $C_1$-$C_4$ et $R_4$ représente un radical alcoyloxy en $C_1$-$C_4$ ainsi que ses formes tautomères lorsqu'elles existent, caractérisé en ce que :

a) on fait réagir une alcoyl($C_1$-$C_4$)oxy-4 phénylhydrazine sur la nitro-2 acétophénone, pour obtenir un alcoyloxy-5 (nitro-2 phényl)-2 indole de formule :

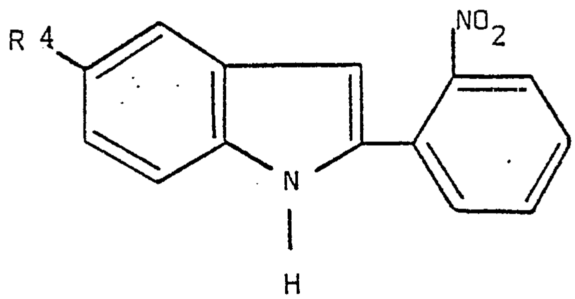

IVa

dans laquelle $R_4$ est un groupe alcoyloxy en $C_1$-$C_4$,

b) on fait réagir sur le composé IVa un halogénure $R'_1$-hal $R'_1$ étant un groupe alcoyle en $C_1$-$C_4$ ou le groupe tétrahydropyranne-2-yle, hal étant le chlore, le brome ou l'iode, pour préparer un composé de formule IVb,

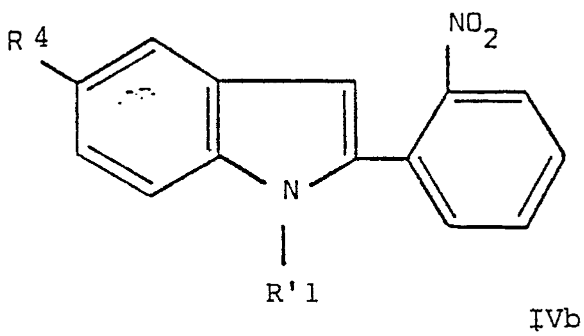

IVb

dans laquelle $R'_1$ représente un groupe alcoyle en $C_1$-$C_4$ ou le groupe tétrahydropyranne-2-yle et $R_4$ un groupe alcoxy en $C_1$-$C_4$,

c) on effectue la réduction catalytique du composé de formule IVb pour obtenir un dérivé aminé de formule V,

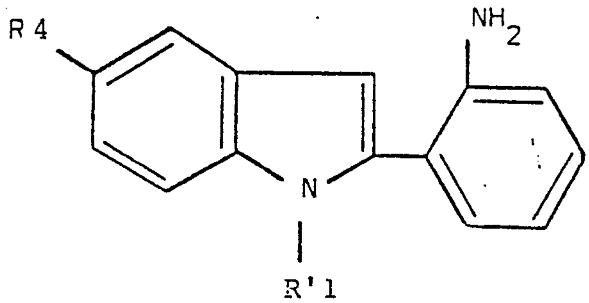

V

dans laquelle les radicaux $R'_1$ et $R_4$ ont les mêmes significations que dans la formule IVb,

d) on fait réagir un chloroformiate d'alcoyle en $C_1$-$C_4$ sur le composé de formule V pour obtenir le carbamate de formule :

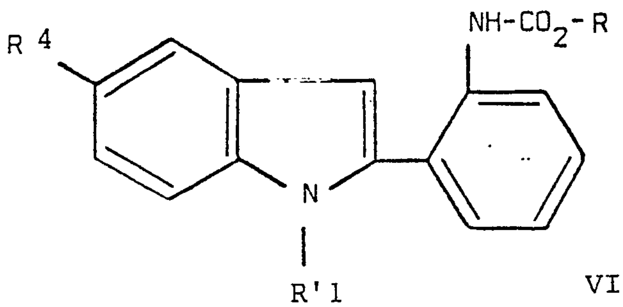

VI

dans laquelle R représente un radical alcoyle en $C_1$-$C_4$ et $R'_1$ et $R_4$ ont les mêmes significations que dans la formule IVb,

e) on soumet à la chaleur le carbamate de formule VI, pour obtenir par cyclisation une 5-H, 11-H, indolo[3,2-c] quinolone-6 de formule :

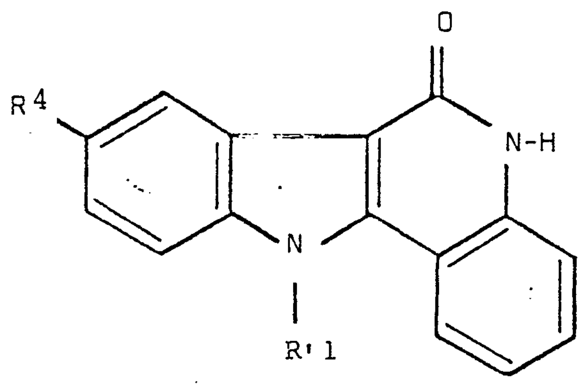

VII

dans laquelle $R'_1$ et $R_4$ ont les mêmes significations que dans la formule IVb,

f) on fait réagir un agent chlorurant sur le composé de formule VII pour obtenir le composé de formule III dans laquelle $R_1$ représente soit l'hydrogène lorsque dans le composé de formule VII mis en oeuvre le radical $R'_1$ était le groupe tétrahydropyranne-2-yle, soit un radical alcoyle en $C_1$-$C_4$.

6. Procédé selon la revendication 5, caractérisé en ce que la formation du composé de formule IVb s'effectue à une température comprise entre 0° C et 80° C dans un solvant inerte aprotique tel que le N,N-diméthylformamide.

7. Procédé selon la revendication 5 ou 6, caractérisé en ce que la réduction du composé de formule IVb est effectuée par action de $H_2$ sous pression ordinaire à une température comprise entre 20° C et 60° C en présence d'un catalyseur tel que le charbon palladié.

8. Procédé selon l'une quelconque des revendications 5 à 7, caractérisé en ce que le carbamate de formule VI est obtenu par action d'un chloroformiate d'alcoyle à une température comprise entre 40° C et 80° C en présence d'une base telle que la pyridine.

9. Procédé selon l'une quelconque des revendications 5 à 8, caractérisé en ce que la cyclisation du composé de formule VI est réalisée à une température comprise entre 150° C et 210° C dans un solvant inerte tel que l'éther diphénylique.

10. Procédé selon l'une quelconque des revendications 5 à 9, caractérisé en ce que la chloration du composé de formule VII s'effectue par action de l'oxychlorure de phosphore porté au reflux.

Office européen des brevets

# RAPPORT DE RECHERCHE EUROPEENNE

Numéro de la demande

EP 86 40 2676

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | FR-A- 913 931 (RHONE-POULENC)<br>* Page 1, lignes 1-14 *<br>--- | 1,9 | C 07 D 471/04<br>A 61 K 31/435//<br>(C 07 D 471/04<br>C 07 D 221:00<br>C 07 D 209:00 ) |
| P,A | EP-A-0 197 829 (SANOFI)<br>* Revendications 1,10 *<br>----- | 1,9 | |
| | | | **DOMAINES TECHNIQUES RECHERCHES (Int. Cl.4)** |
| | | | C 07 D 471/00<br>A 61 K 31/00 |

Le présent rapport de recherche a été établi pour toutes les revendications

| Lieu de la recherche | Date d'achèvement de la recherche | Examinateur |
|---|---|---|
| LA HAYE | 10-03-1987 | ALFARO I. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention
E : document de brevet antérieur, mais publié à la date de dépôt ou après cette date
D : cité dans la demande
L : cité pour d'autres raisons
& : membre de la même famille, document correspondant

OEB Form 1503 03 82